# DEMANDE DE BREVET EUROPEEN

(11) **EP 4 840 439 A1**
(43) Date de publication de la demande: **18.11.2026**
(21) Numéro de dépôt: 26178590.1
(22) Date de dépôt: 13.05.2026
(51) Int. Cl.: A61K 8/34, A61K 8/06, A61K 8/36, A61K 8/37, A61K 8/60, A61Q 17/00, A61Q 19/00, A61K 8/73, A61K 31/7024, A61K 31/728, A61K 47/00, A61P 17/00, A61P 31/04, A61K 9/107, A61K 47/36, A61K 47/38

(54) **BASE DE FORMULATION COSMÉTIQUE, PHARMACEUTIQUE, OU DE DISPOSITIF MÉDICAL AYANT UNE ACTIVITÉ BACTÉRICIDE ET UNE TOLÉRANCE CUTANÉE AMÉLIORÉE**

(30) Priorité: 16.05.2025 FR 2505244
(71) Demandeur: Laboratoires Chemineau, 37210 Vouvray (FR)
(72) Inventeur: LAFARGUE, David, 47200 Marmande (FR); BERTIN, Marie, 37700 La Ville aux Dames (FR); BERNARD, Fabrice, 37250 Montbazon (FR); MOUSSU, Alexandra, 37320 Saint-Branchs (FR)
(74) Mandataire: Yes My Patent

(57) **Abrégé**

L'invention concerne une base de formulation cosmétique, pharmaceutique ou de dispositif médical, sous forme d'une émulsion gélifiée huile dans eau destinée à être utilisée ultérieurement avec un ou plusieurs principe actif d'indication, comprenant de l'eau, de la glycérine, des triglycérides d'acide caprylique et d'acide caprique, au moins un tensioactif, au moins un gélifiant, du pentylène glycol et/ou du sorbate de potassium, ladite base présentant une tolérance cutanée améliorée et une activité bactéricide.

## Description

L'invention concerne une base de formulation cosmétique, pharmaceutique ou de dispositif médical, sous forme d'une émulsion gélifiée huile dans eau destinée à être utilisée ultérieurement avec un ou plusieurs actifs, comprenant de l'eau, de la glycérine, des triglycérides d'acide caprylique et d'acide caprique, au moins un tensioactif, au moins un gélifiant, du pentylène glycol et/ou du sorbate de potassium, ladite base présentant une tolérance cutanée améliorée et une activité bactéricide.

### Art Antérieur

Les tensioactifs sont des molécules amphiphiles qui sont utilisées dans de très nombreux secteurs industriels (Cosmétiques, Pharmaceutiques, Dispositif médicaux, Agroalimentaires, Chimies, hygiènes...) en raison de leurs propriétés émulsifiantes et détergentes. On compte aujourd'hui plus de 5000 tensioactifs commercialisés mais seulement un petit nombre d'entre eux répond aux critères de tolérance indispensables à une utilisation cosmétique (Martini, M. C. 2006. Introduction a la dermopharmacie et a la cosmetologie. Tec & Doc ; Ca-chan : Ed. Medicales internationales, 2eme edition, Paris ; Londres ; New York, 448 pp) . Leurs propriétés émulsifiantes se révèlent essentielles pour stabiliser les émulsions. Dans les produits dermo-cosmétiques, leurs propriétés détergentes se révèlent un atout majeur pour nettoyer la peau mais se révèlent être également la cause de nombreux troubles cutanés lors d'exposition prolongée à des concentrations élevées (E. Lémery et al., Skin toxicity of surfactants: Structure/toxicity relationships, Colloids and Surfaces A: Physicochemical and Engineering Aspects, Volume 469, Pages 166-179 (2015) & E. Bárány et al. Un-expected skin barrier influence from nonionic emulsifiers, Int. J. Pharm. Volume 195, Issues 1-2, Pages 189-195 (2000)).

De par leurs propriétés permettant de solubiliser les corps gras, les tensioactifs interagissent avec les composants de la peau en perturbant leur fonctionnement (dénaturation des kératinocytes, solubilisations des lipides du stratum cornéum, modification de la barrière cutanée, perturbation du microbiote...). Une des premières preuves évidentes de l'interaction des tensioactifs avec la peau est l'observation des signes cliniques liés à une exposition prolongée ou chronique à des formules riches en tensioactifs. Sensation de tiraillement, rugosité, irritations, dessèchement, sont connues pour engendrer des dermatites irritatives de contact (M.N. Crepy,Dermatoses Professionelles Aux Detergents, Fiche d'aller-gologiedermatologie professionelle, , no. 103: 375-84, 2005). Willis et al. ont démontré que les molécules de tensioactifs pouvaient agir sur les membranes cellulaires, et être responsables de la libération de cytokines provoquant la pro-liferation cellulaire (Willis, Stephens, and Wilkinson 1992). En effet l'interaction des tensioactifs sur les kératinocytes est connue pour produire des médiateurs de l'inflammation, les cytokines. L'IL-1α est aussi libérée naturellement suite à une perturbation de la fonction barrière, faisant partie du processus de protection visant à stimuler la réparation de la barrière (C. M. Willis et al. "Epi-dermal Damage Induced by Irritants in Man: A Light and Electron Microscopic Study." Journal of Investigative Dermatology 93 (5): 695-99, (1989)).

L'impact des tensioactifs sur la toxicité cutanée et la fonction barrière a fait l'objet de très nombreux travaux qui mettent en évidence le rôle la charge ionique de ces molécules sur ces interactions avec la peau(E. Lemery, Eur J Dermatol , Sep-Oct;25(5):424-35 (2015)).

S'il est reconnu depuis de nombreuses années que les tensioactifs non-ioniques sont en général moins toxiques que les anioniques, cationiques ou amphotères (T. Fujimura T et al. Changes in hydration of the stratum corneum are the most suitable indica- tor to evaluate the irritation of surfactants on the skin. Skin Res Technol. 2017;23(1):97-103) quelques études récentes mettent en évidence des effets délétères sur le fonctionnement de la barrière cutanée (E. Lemery et al. Surfactants have multi-fold effects on skin barrier function, Eur J Dermatol Sep-Oct;25(5):424-35 (2015)).

Les crèmes et lotions conventionnelles contiennent généralement de 2 à 10 % de tensioactifs pour maintenir la stabilité physique des émulsions. La quantité de tensioactif qui provoque une irritation telle qu'un érythème ou un changement mesurable de l'hydratation de la peau peut être aussi faible que 0,5 % pour les tensioactifs anioniques et dès 2 % p/p (KP Wilhelm et al. Effect of sodium lauryl sulfate-induced skin irritation on in vivo percutaneous penetration of four drugs. J Invest Dermatol;97(5):927-32(1991)) pour les tensioactifs non ioniques.

Les émulsions dermo-cosmétiques étant utilisées pour prévenir ou traiter des troubles cutanées voir des pathologies inflammatoires de la peau, il est primordial de pouvoir développer des alternatives offrant une meilleure tolérance pour la peau.

Face aux limitations de l'art antérieur, la présente invention vise à fournir une base de formulation qui soit non seulement stable et très bien tolérée par la peau, mais qui présente également une activité bactéricide intrinsèque permettant un meilleur contrôle du microbiote cutané. Ce problème est résolu par la base de formulation selon la revendication 1.

Le Demandeur a développé une base de formulation sous forme d'émulsion gélifiée huile dans eau, d'origine naturelle, dont la teneur en tensioactif est très faible, en vue d'améliorer la tolérance locale sur la peau. Cette base de formulation démontre également un effet antibactérien.

Cette base de formulation comprend :
- une teneur faible en tensioactif < 1% ;
- une concentration supérieure à 98% d'ingrédients d'origine naturelle ;
- un pH cible entre 5 et 5.5 ;
- une taille de globules inférieur à 20 µm et un tapis globulaire homogène.

### Description détaillée de l'invention

Selon un premier aspect, l'invention concerne une base de formulation cosmétique ou pharmaceutique, sous forme d'une émulsion gélifiée huile dans eau, comprenant :
- De l'eau,
- De la glycérine ;
- Des triglycérides d'acide caprylique et d'acide caprique ;
- Au moins un tensioactif ;
- Au moins un gélifiant ;
- Du pentylène glycol et/ou du sorbate de potassium ;
   ladite base présentant une tolérance cutanée améliorée et une activité bactéricide.

L'invention concerne une base de formulation cosmétique ou pharmaceutique, sous forme d'une émulsion gélifiée huile dans eau, destinée à être mélangée ultérieurement avec un ou plusieurs principes actifs d'indication cosmétique ou pharmaceutique.

La base de formulation selon l'invention est une émulsion gélifiée huile dans eau, d'origine naturelle, dont la teneur en tensioactif est très faible, de préférence inférieure à 1%, permettant d'améliorer la tolérance locale sur la peau ou les muqueuses nasale, buccale, vaginale, ou ophtalmique.

La base de formulation cosmétique ou pharmaceutique, selon l'invention a une stabilité microbiologique d'au moins 6 mois à température ambiante sans ajout de conservateur supplémentaire.

Selon un mode de réalisation, la base de formulation cosmétique ou pharmaceutique selon l'invention comprend de l'acide citrique.

Selon un mode de réalisation, la base de formulation cosmétique ou pharmaceutique selon l'invention comprend de l'eau, de préférence en quantité suffisante pour compléter la base de formulation.

Selon un mode de réalisation, la base de formulation cosmétique ou pharmaceutique selon l'invention a un pH compris entre 5,0 et 5,5. Ce pH est obtenu en ajoutant de l'acide citrique en quantité efficace.

La base de formulation cosmétique ou pharmaceutique sous forme d'une émulsion gélifiée huile dans eau, a une taille de globules inférieur à 20 µm et un tapis globulaire homogène en terme de taille et de forme des globules de la phase huileuse.

Selon un mode de réalisation, la base de formulation cosmétique ou pharmaceutique selon l'invention comprend entre 3% et 8% de glycérine, de préférence 5%, en poids total de ladite base de formulation.

Selon un mode de réalisation, la base de formulation cosmétique ou pharmaceutique selon l'invention comprend 3 % ou 3,25 % ou 3,5 % ou 3,75 % ou 4 % ou 4,25 % ou 4,5 % ou 4,75 % ou 5 % ou 5,25 % ou 5,5 % ou 5,75 % ou 6 % ou 6,25 % ou 6,5 % ou 6,75 % ou 7 % ou 7,25 % ou 7,5 % ou 7,75 % ou 8 % de glycérine, de préférence 5%, en poids total de ladite base de formulation.

Selon un mode de réalisation, la base de formulation cosmétique ou pharmaceutique selon l'invention comprend entre 10 et 30% de triglycérides d'acide caprylique et d'acide caprique, de préférence 20%, en poids total de ladite base de formulation.

Selon un mode de réalisation, la base de formulation cosmétique ou pharmaceutique selon l'invention comprend 10 % ou 10,5 % ou 11 % ou 11,5 % ou 12 % ou 12,5 % ou 13 % ou 13,5 % ou 14 % ou 14,5 % ou 15 % ou 15,5 % ou 16 % ou 16,5 % ou 17 % ou 17,5 % ou 18 % ou 18,5 % ou 19 % ou 19,5 % ou 20 % ou 20,5 % ou 21 % ou 21,5 % ou 22 % ou 22,5 % ou 23 % ou 23,5 % ou 24 % ou 24,5 % ou 25 % ou 25,5 % ou 26 % ou 26,5 % ou 27 % ou 27,5 % ou 28 % ou 28,5 % ou 29 % ou 29,5 % ou 30 %de triglycérides d'acide caprylique et d'acide caprique, de préférence 20%, en poids total de ladite base de formulation.

Selon un mode de réalisation, le ratio entre acide caprylique et acide caprique dans les triglycérides d'acide caprylique et d'acide caprique est compris entre 60:40 et 80:20, de préférence 70:30, en faveur de l'acide caprylique.

Selon un mode de réalisation, ledit au moins un tensioactif est choisi parmi :
- Un mélange de lauryl glucoside, de myristyl glucoside et de polyglyceryl-6-laurate.
- Un mélange d'oléate de polyglycéryle-6 et d'olivate de potassium ;
- Du polyglyceryl-2-dipolyhydroxystearate ;
- Du polyglyceryl-3-diisostearate ;
- Des phospholipides hydrogénés ;
- Du myristate de polyglycéryle-10 ;
- Du surfactine sodique ;
- Un mélange d'alcool cétéarylique et de glucoside de cétéaryle ;
- Des extraits de graines de soja ;
- De la lécithine ;
- Du polysorbate 80 ;
- De l'oléate de sorbitan.

Selon un mode de réalisation, la base de formulation cosmétique ou pharmaceutique selon l'invention comprend entre 0,1 % et 1 % en poids total de ladite base de formulation dudit au moins un tensioactif.

Selon un mode de réalisation, la base de formulation cosmétique ou pharmaceutique selon l'invention comprend 0,1 % ou 0,15 % ou 0,2 % ou 0,25 % ou 0,3 % ou 0,35 % ou 0,4 % ou 0,45 % ou 0,5 % ou 0,55 % ou 0,6 % ou 0,65 % ou 0,7 % ou 0,75 % ou 0,8 % ou 0,85 % ou 0,9 % ou 0,95 % ou 1 % en poids total de ladite base de formulation dudit au moins un tensioactif.

Selon un mode de réalisation, ledit au moins un tensioactif est un mélange de lauryl glucoside, de myristyl glucoside et de polyglyceryl-6-laurate.

Selon un mode de réalisation, la base de formulation cosmétique ou pharmaceutique selon l'invention comprend entre 0,024 et 0,28% de lauryl glucoside, de préférence entre 0,12% et 0,14%, en poids total de ladite base de formulation cosmétique ou pharmaceutique.

Selon un mode de réalisation, la base de formulation cosmétique ou pharmaceutique selon l'invention comprend 0,024 % ou 0,030 % ou 0,035 % ou 0,040 % ou 0,045 % ou 0,050 % ou 0,055 % ou 0,060 % ou 0,065 % ou 0,070 % ou 0,075 % ou 0,080 % ou 0,085 % ou 0,090 % ou 0,095 % ou 0,100 % ou 0,105 % ou 0,110 % ou 0,115 % ou 0,120 % ou 0,125 % ou 0,130 % ou 0,135 % ou 0,140 % ou 0,145 % ou 0,150 % ou 0,155 % ou 0,160 % ou 0,165 % ou 0,170 % ou 0,175 % ou 0,180 % ou 0,185 % ou 0,190 % ou 0,195 % ou 0,200 % ou 0,205 % ou 0,210 % ou 0,215 % ou 0,220 % ou 0,225 % ou 0,230 % ou 0,235 % ou 0,240 % ou 0,245 % ou 0,250 % ou 0,280 % de lauryl glucoside en poids total de ladite base de formulation.

Selon un mode de réalisation, la base de cosmétique ou pharmaceutique selon l'invention comprend entre 0,01 et 0,12% de myristyl glucoside, de préférence entre 0,05% et 0,06%, en poids total de ladite base de formulation cosmétique ou pharmaceutique.

Selon un mode de réalisation, la base de formulation cosmétique ou pharmaceutique selon l'invention comprend 0,010 % ou 0,015 % ou 0,020 % ou 0,025 % ou 0,030 % ou 0,035 % ou 0,040 % ou 0,045 % ou 0,050 % ou 0,055 % ou 0,060 % ou 0,065 % ou 0,070 % ou 0,075 % ou 0,080 % ou 0,085 % ou 0,090 % ou 0,095 % ou 0,100 % ou 0,105 % ou 0,110 % ou 0,115 % ou 0,120 % de myristyl glucoside, en poids total de ladite base de formulation.

Selon un mode de réalisation, la base de formulation cosmétique ou pharmaceutique selon l'invention comprend entre 0,0235 % et 0,245% de polyglyceryl-6-laurate, de préférence entre 0,1175% et 0,1225, en poids total de ladite base de formulation.

Selon un mode de réalisation, la base de formulation cosmétique ou pharmaceutique selon l'invention comprend 0,0235 % ou 0,0300 % ou 0,0350 % ou 0,0400 % ou 0,0450 % ou 0,0500 % ou 0,0550 % ou 0,0600 % ou 0,0650 % ou 0,0700 % ou 0,0750 % ou 0,0800 % ou 0,0850 % ou 0,0900 % ou 0,0950 % ou 0,1000 % ou 0,1050 % ou 0,1100 % ou 0,1150 % ou 0,1200 % ou 0,1250 % ou 0,1300 % ou 0,1350 % ou 0,1400 % ou 0,1450 % ou 0,1500 % ou 0,1550 % ou 0,1600 % ou 0,1650 % ou 0,1700 % ou 0,1750 % ou 0,1800 % ou 0,1850 % ou 0,1900 % ou 0,1950 % ou 0,2000 % ou 0,2050 % ou 0,2100 % ou 0,2150 % ou 0,2200 % ou 0,2250 % ou 0,2300 % ou 0,2350 % ou 0,2400 % ou 0,2450 % de polyglyceryl-6-laurate en poids total de ladite base de formulation.

Selon un mode de réalisation, la base de formulation cosmétique ou pharmaceutique selon l'invention comprend 0,1 % ou 0,15 % ou 0,2 % ou 0,25 % ou 0,3 % ou 0,35 % ou 0,4 % ou 0,45 % ou 0,5 % ou 0,55 % ou 0,6 % ou 0,65 % ou 0,7 % ou 0,75 % ou 0,8 % ou 0,85 % ou 0,9 % ou 0,95 % ou 1 % d'un mélange d'eau, de lauryl glucoside, de myristyl glucoside et de polyglyceryl-6-laurate, en poids total de ladite base de formulation, ledit mélange comprenant : entre 24 et 28% du mélange de lauryl glucoside, entre 10 et 12% du mélange de myristyl glucoside, entre 23,5 et 24,5 du mélange de polyglyceryl-6 laurate et entre 30 et 45% du mélange d'eau.

Selon un mode de réalisation, ledit au moins un gélifiant est choisi parmi : gomme sclérotique, gomme de xanthane, cellulose microcristalline, agar, carraghénanes, pectines, guar, caroube, arabique, gélatine, collagène, cellulose, amidons, chitosan, alginates, argiles.

Selon un mode de réalisation, la base de formulation cosmétique ou pharmaceutique selon l'invention comprend entre 0,1% et 5% d'au moins un gélifiant, en poids total de ladite base de formulation.

Selon un mode de réalisation, la base de formulation cosmétique ou pharmaceutique selon l'invention comprend 0,1 % ou 0,2 % ou 0,3 % ou 0,4 % ou 0,5 % ou 0,6 % ou 0,7 % ou 0,8 % ou 0,9 % ou 1,0 % ou 1,1 % ou 1,2 % ou 1,3 % ou 1,4 % ou 1,5 % ou 1,6 % ou 1,7 % ou 1,8 % ou 1,9 % ou 2,0 % ou 2,1 % ou 2,2 % ou 2,3 % ou 2,4 % ou 2,5 % ou 2,6 % ou 2,7 % ou 2,8 % ou 2,9 % ou 3,0 % ou 3,1 % ou 3,2 % ou 3,3 % ou 3,4 % ou 3,5 % ou 3,6 % ou 3,7 % ou 3,8 % ou 3,9 % ou 4,0 % ou 4,1 % ou 4,2 % ou 4,3 % ou 4,4 % ou 4,5 % ou 4,6 % ou 4,7 % ou 4,8 % ou 4,9 % ou 5,0 % d'au moins un gélifiant, en poids total de ladite base de formulation.

Selon un mode de réalisation, ledit au moins un gélifiant est choisi parmi :
- Entre 0,2% et 5% de gomme de xanthane en poids total de ladite base de formulation cosmétique, pharmaceutique, ou de dispositif médical ; et/ou
- Entre 2% et 3% d'un mélange de gomme de xanthane et de cellulose microcristalline en poids total de ladite base de formulation cosmétique, pharmaceutique, ou de dispositif médical ; et/ou
- entre 0,1 et 2% de gomme sclérotique, de préférence 1%, en poids total de ladite base de formulation cosmétique, pharmaceutique, ou de dispositif médical.

Selon un mode de réalisation, la base de formulation cosmétique ou pharmaceutique selon l'invention comprend entre 0,1 et 2% de gomme sclérotique, de préférence 1%, en poids total de ladite base de formulation.

Selon un mode de réalisation, la base de formulation cosmétique ou pharmaceutique selon l'invention comprend 0,1 % ou 0,15 % ou 0,2 % ou 0,25 % ou 0,3 % ou 0,35 % ou 0,4 % ou 0,45 % ou 0,5 % ou 0,55 % ou 0,6 % ou 0,65 % ou 0,7 % ou 0,75 % ou 0,8 % ou 0,85 % ou 0,9 % ou 0,95 % ou 1 % ou 1,05 % ou 1,1 % ou 1,15 % ou 1,2 % ou 1,25 % ou 1,3 % ou 1,35 % ou 1,4 % ou 1,45 % ou 1,5 % ou 1,55 % ou 1,6 % ou 1,65 % ou 1,7 % ou 1,75 % ou 1,8 % ou 1,85 % ou 1,9 % ou 1,95 % ou 2 % de gomme sclérotique, de préférence 1%, en poids total de ladite base de formulation.

Selon un mode de réalisation, la base de formulation cosmétique ou pharmaceutique selon l'invention comprend en outre entre 0,01 et 0,5% de tocophérol, de préférence 0,3%, en poids total de ladite base de formulation.

Selon un mode de réalisation, la base de formulation cosmétique ou pharmaceutique selon l'invention comprend en outre 0,01 % ou 0,025 % ou 0,05 % ou 0,075 % ou 0,1 % ou 0,125 % ou 0,15 % ou 0,175 % ou 0,2 % ou 0,225 % ou 0,25 % ou 0,275 % ou 0,3 % ou 0,325 % ou 0,35 % ou 0,375 % ou 0,4 % ou 0,425 % ou 0,45 % ou 0,475 % ou 0,5 % de tocophérol, de préférence 0,3%, en poids total de ladite base de formulation.

Selon un mode de réalisation, la base de formulation cosmétique ou pharmaceutique selon l'invention comprend entre 1 et 10% de pentylène glycol, de préférence 5%, en poids total de ladite base de formulation.

Selon un mode de réalisation, la base de formulation cosmétique ou pharmaceutique selon l'invention comprend entre 3% et 7% de pentylène glycol, de préférence 5%, en poids total de ladite base de formulation.

Selon un mode de réalisation, la base de formulation cosmétique ou pharmaceutique selon l'invention comprend 1 % ou 1,5 % ou 2 % ou 2,5 % ou 3 % ou 3,5 % ou 4 % ou 4,5 % ou 5 % ou 5,5 % ou 6 % ou 6,5 % ou 7 % ou 7,5 % ou 8 % ou 8,5 % ou 9 % ou 9,5 % ou 10 % de pentylène glycol, de préférence 5%, en poids total de ladite base de formulation.

Selon un mode de réalisation, la base de formulation cosmétique ou pharmaceutique selon l'invention comprend entre 0,01 et 0,25% de sorbate de potassium, de préférence 0,125%, en poids total de ladite base de formulation.

Selon un mode de réalisation, la base de formulation cosmétique ou pharmaceutique selon l'invention comprend entre 0,1 et 0,15% de sorbate de potassium, de préférence 0,125%, en poids total de ladite base de formulation.

Selon un mode de réalisation, la base de formulation cosmétique ou pharmaceutique selon l'invention comprend 0,01 % ou 0,02 % ou 0,03 % ou 0,04 % ou 0,05 % ou 0,06 % ou 0,07 % ou 0,08 % ou 0,09 % ou 0,10 % ou 0,11 % ou 0,12 % ou 0,13 % ou 0,14 % ou 0,15 % ou 0,16 % ou 0,17 % ou 0,18 % ou 0,19 % ou 0,20 % ou 0,21 % ou 0,22 % ou 0,23 % ou 0,24 % ou 0,25 % de sorbate de potassium, de préférence 0,125%, en poids total de ladite base de formulation.

Selon un mode de réalisation, la base de formulation cosmétique ou pharmaceutique selon l'invention comprend en outre au moins un principe actif d'indication sélectionné parmi un antibiotique, un agent bactéricide, bactériostatique, antifongique, antiseptique, anti-inflammatoire et/ou un corticoïde.

À titre d'exemples non limitatifs, ledit au moins un principe actif d'indication peut être choisi parmi : flocculosine, triclosan, chlorhexidine et ses sels (tels que le digluconate et le diacétate de chlorhexidine), polyhexaméthylène biguanide (PHMB), éthanol, isopropanol, acide salicylique, acide azélaïque, acide succinique, acide lactique, acide benzoïque, benzoate de sodium, acide sor-bique, sorbate de potassium, huiles essentielles (comme l'huile d'arbre à thé (tea tree), l'huile d'origan, l'huile de thym), argent colloïdal, nitrate d'argent, sulfadiazine argentique, piroctone olamine, hexamidine, chlorure de benzalkonium, peroxyde de benzoyle, cetrimonium bromide, zinc pyrithione, lactofer-rine, peptides antimicrobiens (AMPs), endolysines, bactériophages, lysostaphine, miel médical (tel que le miel de Manuka), extraits de propolis, de bardane, de romarin, thymol, eugénol et cinnamaldéhyde.

Il peut également s'agir :
- d'antibiotiques tels que l'amoxicilline, l'ampicilline, la pipéracilline, la céfalexine, le céfuroxime, le céfotaxime, l'érythromycine, l'azithromycine, la clarithromycine, la doxycycline, la minocycline, la gentamicine, l'amika-cine, la ciprofloxacine, la lévofloxacine, la vancomycine, la teicoplanine, l'imipénem et le méropénem ; et/ou
- d'antiseptiques tels que l'alcool éthylique (éthanol), l'alcool isopropylique, le gluconate de chlorhexidine, la povidone iodée, le peroxyde d'hydrogène, le chlorure de benzalkonium, le chlorure de cétrimonium et l'hexachloro-phène ; et/ou
- d'agents antifongiques tels que le clotrimazole, le miconazole, l'éconazole, le kétoconazole, la terbinafine, la nystatine, le ciclopirox, la naftifine, l'oxi-conazole et le sertaconazole ; et/ou
- de corticoïdes tels que le propionate de clobétasol, le propionate d'halobéta-sol, le dipropionate de bétaméthasone, la désoximétasone, le fluocinonide, l'acétonide de triamcinolone, l'amcinonide, le butyrate de clobétasone, le fu-roate de mométasone, le valérate de bétaméthasone, le prednicarbate, le dé-sonide et l'hydrocortisone ; et/ou
- d'anti-inflammatoires tels que l'acide acétylsalicylique (aspirine), l'ibupro-fène, le naproxène, le diclofénac, l'indométhacine, le kétoprofène, le méloxi-cam, le piroxicam, le sulindac et le célécoxib ; et/ou
- d'autres agents thérapeutiques tels que la clindamycine, le métronidazole, la trétinoïne, l'adapalène, le tacrolimus, le pimécrolimus, l'ivermectine, l'aci-trétine, l'ustékinumab ou le sécukinumab.

Un exemple de base de formulation selon l'invention est décrite en tableau 1.

**[Table 1]**

| **Composés** | **Fonction** | **Base selon l'invention (en % m/m)** |
|---|---|---|
| **Eau purifiée** | **Solvant** | **Qs 100** |
| **Glycérine** | **Agent humectant** | **5.00** |
| **Triglycéride d'acide caprylique et d'acide caprique;** | **Solvant huileux** | **20.00** |
| **Lauryl glucoside, myristyl glucoside et polyglyceryl-6-laurate** | **Emulsionnant** | **0.50** |
| **Gomme sclérotique** | **Agent gélifiant** | **1.00** |
| **Tocopherol** | **Anti-oxydant** | **0.30** |
| **Pentylène glycol** | **Conservateur** | **5.00** |
| **Sorbate de potassium** | **Conservateur** | **0.125** |
| **Acide citrique** | **Ajusteur de pH** | **Qs pH 5.0-5.5** |
| | **Total** | **100** |

Selon un mode de réalisation, la base de formulation cosmétique ou pharmaceutique selon l'invention comprend ou consiste en :
- 5% glycérine, en poids total de ladite base de formulation ;
- 20% de triglycérides d'acide caprylique et d'acide caprique, en poids total de ladite base de formulation ;
- 0,50% d'un mélange d'eau, de lauryl glucoside, de myristyl glucoside et de polyglyceryl-6-laurate, en poids total de ladite base de formulation, ledit mélange comprenant : entre 24 et 28% du mélange de lauryl glucoside, entre 10 et 12% du mélange de myristyl glucoside, entre 23,5 et 24,5 du mélange de polyglyceryl-6 laurate et entre 30 et 45% du mélange d'eau ;
- 1% de gomme sclérotique, en poids total de ladite base de formulation ;
- 5% de pentylène glycol, en poids total de ladite base de formulation ;
- 0,125% de sorbate de potassium, en poids total de ladite base de formulation ;
- De l'eau en quantité suffisante pour compléter la base de formulation ;
- De l'acide citrique en quantité suffisante pour obtenir un pH compris entre 5 et 5,5.

Des exemples de formulation comprenant la base de formulation selon l'invention sont décrites en tableau 2. Des principes actifs d'indication ont été ajoutés afin d'obtenir des formules dermo-cosmétique destinées au traitement de l'acné et de la dermatite atopique. Ces deux compositions se différencient par la présence d'actifs spécifiques : un mélange de pré/postbiotique (ECOSKIN^{®}) et d'acide hyaluronique (BASHYAL POUDRE^{®}) pour l'acné et un actif antibactérien ciblé (SERENIBIOME^{®}) qui est un mélange de propanediol et de glycolipides pour la dermatite atopique.

**[Table 2]**

| **Composés** | **Fonction** | **Formule « acné » (en % m/m)** | **Formule « dermatite » (en % m/m)** |
|---|---|---|---|
| **Eau purifiée** | **Solvant** | **Qs 100** | **Qs 100** |
| **Glycérine** | **Agent humectant** | **5.00** | **5.00** |
| **Triglycéride d'acide caprylique et d'acide caprique;** | **Solvant huileux** | **20.00** | **20.00** |
| **Lauryl glucoside, myristyl glucoside et polyglyceryl-6-laurate** | **Emulsionnant tensioactif** | **0.50** | **0.50** |
| **Gomme sclérotique** | **Agent gélifiant** | **1.00** | **1.00** |
| **Tocopherol** | **Anti-oxydant** | **0.30** | **0.30** |
| **Pentylène glycol** | **Conservateur** | **5.00** | **5.00** |
| **Sorbate de potassium** | **Conservateur** | **0.125** | **0.125** |
| **Acide citrique** | **Ajusteur de pH** | **Qs pH 5.0 -5.5** | **Qs pH 5.0 -5.5** |
| **Propanediol & glycolypides (Serenibiome ^{®})** | **Actif** | **-** | **2.00** |
| **Oligosaccharide d'alpha-glucane (et) Jus de racine de Polymnia sonchifolia (et) Maltodextrine (et) Lactobacillus (Ecoskin ^{®})** | **Actif** | **1.00** | **-** |
| **Hyaluronate de sodium (Bashyal^{®})** | **Actif** | **0.25** | **-** |
| **Hyaluronate de sodium Primahyal^{®} 50** | **Actif** | **0.25** | |
| | **Total** | **100** | **100** |

Dans les tableaux, le terme « Lauryl glucoside, myristyl glucoside et polyglyceryl-6-laurate » désigne un mélange d'eau, de lauryl glucoside, de myristyl glucoside et de polyglyceryl-6-laurate, en poids total de ladite base de formulation cosmétique ou pharmaceutique, ledit mélange comprenant : entre 24 et 28% du mélange de lauryl glucoside, entre 10 et 12% du mélange de myristyl glucoside, entre 23,5 et 24,5 du mélange de polyglyceryl-6 laurate et entre 30 et 45% du mélange d'eau.

Selon un autre aspect, l'invention concerne l'utilisation de la base de formulation cosmétique ou pharmaceutique selon l'invention, pour la préparation d'une formulation cosmétique ou pharmaceutique contenant au moins un principe actif d'indication pharmaceutique ou cosmétique.

Selon un autre aspect, l'invention concerne un dispositif médical comprenant une base de formulation selon l'invention, ladite base de formulation étant destinée à être mélangée avec au moins un principe actif d'indication cosmétique ou pharmaceutique.

Selon un mode de réalisation, ledit dispositif médical est sélectionné parmi :
- un dispositif topique (par exemple pansement, patch, compresse) ;
- un dispositif injectable (par exemple seringue préremplie, implant injectable) ;
- un dispositif implantable (par exemple implant biodégradable, stent, cathéter) ;
- un dispositif ophtalmique, nasal, auriculaire ou vaginal ;
- un dispositif gastro-intestinal ou buccal.

Selon un mode de réalisation, le dispositif médical selon l'invention est choisi parmi les pansements, compresses, films, patchs transdermiques ou gels destinés à une application cutanée ou muqueuse, les dispositifs injectables comprenant par exemple des seringues préremplies ou des formulations stériles pour administration parentérale, les dispositifs implantables tels que les implants biodégradables, stents, cathéters, valves ou réservoirs à libération prolongée, ainsi que les dispositifs destinés à une administration ophtalmique, auriculaire, nasale, buccale, vaginale ou rectale, tels que des gouttes, inserts, sprays, films orodispersibles, ovules ou suppositoires. La base de formulation peut également être intégrée dans des dispositifs gastro-intestinaux, notamment des capsules, comprimés ou systèmes d'administration ciblée. De manière générale, elle peut être utilisée dans tout dispositif médical destiné à administrer un ou plusieurs principes actifs, localement ou systématiquement, chez un patient humain ou animal.

Selon un mode de réalisation, le dispositif médical selon l'invention est destiné à délivrer localement au moins un principe actif d'indication, par diffusion, résorption ou libération contrôlée.

Selon un autre aspect, l'invention concerne l'utilisation de la base de formulation selon l'invention, pour la fabrication d'un dispositif médical destiné à l'administration d'un principe actif d'indication à un patient humain ou animal.

Selon un autre aspect, l'invention concerne une méthode de préparation d'une composition cosmétique, ou d'une composition pharmaceutique, comprenant au moins une étape de mélange de la base de formulation selon l'invention, avec au moins un principe actif d'indication cosmétiquement ou pharmaceutiquement acceptable.

Selon un autre aspect, l'invention concerne un kit destiné à constituer un dispositif médical, comprenant :
- une base de formulation cosmétique ou pharmaceutique selon l'invention, et
- au moins un compartiment ou contenant séparé destiné à recevoir un ou plusieurs principes actifs d'indication, à mélanger avec ladite base de formulation selon l'invention préalablement à l'administration.

Selon un autre aspect, l'invention concerne un kit cosmétique comprenant :
- une base de formulation cosmétique selon l'invention, et
- un ou plusieurs principe actif d'indication cosmétiques présentés séparément, en vue d'un mélange extemporané.

Selon un autre aspect, l'invention concerne un kit pharmaceutique comprenant :
- une base de formulation pharmaceutique selon l'invention, et
- un ou plusieurs principes actif d'indication pharmaceutique présentés séparément, en vue d'un mélange extemporané.

Selon un autre aspect, l'invention concerne un procédé d'amélioration de la tolérance cutanée et/ou de la stabilité d'un principe actif d'indication cosmétique ou pharmaceutique, comprenant l'incorporation dudit principe actif d'indication dans une base de formulation selon l'invention.

Selon un autre aspect, l'invention concerne une composition cosmétique, comprenant :
- une base de formulation selon l'invention, et
- au moins un principe actif d'indication cosmétique choisi parmi : agents hydratants, humectants, apaisants, antioxydants, matifiants, dépigmentants, éclaircissants, tenseurs, raffermissants, anti-âge, exfoliants doux, agents filmogènes, protecteurs cutanés, agents purifiants, agents tonifiants, agents rafraîchissants, agents calmants, boosters de collagène, agents favorisant le renouvellement cellulaire, réparateurs de la barrière cutanée, actifs d'origine végétale, prébiotiques, probiotiques, peptides biomimétiques, filtres UV, agents séborégulateurs, agents hypoallergéniques, endolysines, bactériophages, agents agissant sur le film hydrolipidique et la barrière cutanée, composés à potentiel apaisant ou réducteur de rougeurs. ou tout autre actif cosmétiquement acceptable selon l'effet recherché.

Selon un autre aspect, l'invention concerne une composition pharmaceutique, comprenant :
- une base de formulation selon l'invention, et
- au moins un principe actif d'indication pharmaceutique choisi parmi : anti-inflammatoires, antibactériens, antifongiques, antiviraux, antiprurigineux, anesthésiques locaux, analgésiques, antiseptiques, agents cicatrisants, immu-nomodulateurs, kératolytiques, agents kératoplastiques, agents antiproliféra-tifs, corticostéroïdes, agents antifibrotiques, agents vasoprotecteurs, agents antihistaminiques, agents anti-acnéiques, agents anti-eczéma, agents antipso-riasiques, agents antirosacée, agents régulateurs de la différenciation cellulaire, agents favorisant la reconstitution de la barrière cutanée, agents modulateurs de l'inflammation cutanée, agents hydratants à visée thérapeutique, agents réparateurs à visée dermatologique, ou tout autre principe actif d'indication pharmaceutique acceptable.

Selon un autre aspect, l'invention concerne un procédé de préparation de la base de formulation cosmétique ou pharmaceutique selon l'invention, comprenant :
- a) Au moins une étape de préparation de la phase aqueuse, comprenant le mélange de l'eau, du gélifiant, de préférence la gomme sclérotique, et du glycérol ;
- b) Au moins une étape de préparation de la phase huileuse, comprenant le mélange des triglycérides d'acide caprylique et d'acide caprique, et du au moins un tensioactif, de préférence un mélange d'eau, de lauryl glucoside, de myristyl glucoside et de le polyglyceryl-6 laurate ;
- c) Au moins une étape d'émulsion de la phase huileuse dans la phase aqueuse ;
- d) Au moins une étape de mélange du potassium sorbate et/ou du pentylène glycol avec l'émulsion obtenue à l'étape c)
- e) Optionnellement, au moins une étape de mélange du tocophérol avec le mélange obtenu à l'étape d)
- f) Optionnellement, ajouter de l'eau et de l'acide citrique afin d'ajuster le pH entre 5 et 5,5.

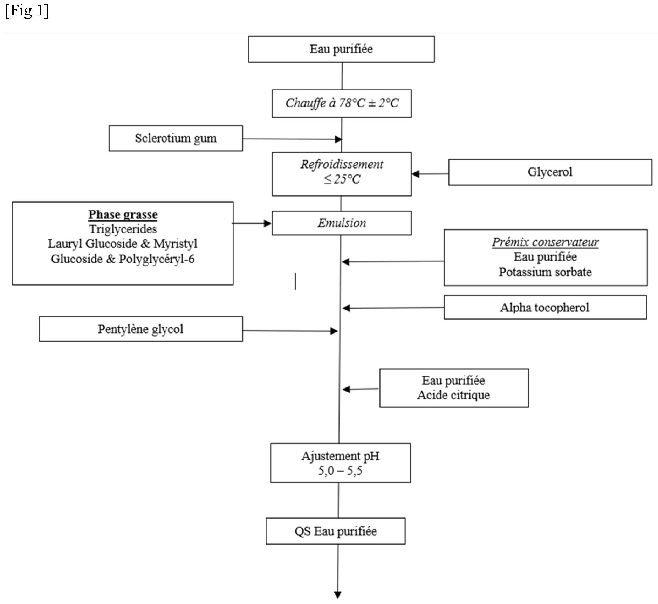

Un exemple des différentes étapes de conception des bases de formulation selon l'invention est décrit dans la figure 1.

Un exemple de conception de la base de formulation est le suivant :
- Etape 1 - Phase aqueuse : Dans un mélangeur : Incorporation eau purifiée puis chauffe à entre 76 et 80°C sous agitation cadre, de préférence 80 tr/min.
- Etape 2 : Incorporation du gélifiant, de préférence la gomme sclérotique : Mélange entre 10 et 20 minutes, de préférence pendant 15 min, sous agitation cadre, de préférence 80 tr/mn et turbine 1400 tr/min. Cette étape est réalisée à entre 76 à 80°C pour obtenir un aspect : gel épais, trouble, bullé, homogène
- Etape 3 : Incorporation de la glycérine : Mélange jusqu'à dissolution complète sous agitation cadre, de préférence 100 tr/min, pendant entre 5 et 15 minutes, de préférence 10 minutes. L'aspect reste ici identique à l'étape précédente.
- Etape 4 : Refroidissement ≤ 25°C sous agitation cadre, de préférence 100 tr/min. L'aspect reste ici identique à l'étape précédente.
- Etape 5 - Phase huileuse : Incorporer les triglycérides d'acide caprylique et d'acide caprique, et ledit au moins un tensioactif non ionique, de préférence un mélange de lauryl glucoside, de myristyl glucoside et de polyglyceryl-6 laurate : Homogénéiser sous agitation turbine, de préférence 600 tr/min, pendant entre 5 et 15 minutes, de préférence 10 minutes. L'aspect est trouble, homogène, fluide.
- Etape 6 : étape d'émulsion : Incorporation de la phase huileuse sous agitation cadre, de préférence à 80 tr/mn à 4400 tr/min, pendant entre 5 et 15 minutes, de préférence 10 minutes. Les phases aqueuses et grasses sont incorporées à une température inférieure à 25°C. L'aspect obtenu ici est une crème blanche, brillante et homogène.
- Etape 7 : Préparation du premix conservateur : Incorporer eau + potassium sorbate - Mélanger jusqu'à dissolution, pendant entre 3 et 8 minutes, de préférence 5 minutes. L'aspect est limpide, incolore et homogène.
- Etape 8 : Incorporation du premix conservateurs et du pentylène glycol : Mélange jusqu'à dissolution complète sous agitation cadre, de préférence 100 tr/mn, pendant entre 3 et 8 minutes, de préférence 5 minutes, à température inférieure à 25°C. L'aspect est celui d'une crème blanche, brillante et homogène.
- Etape 9 : Incorporation des principe actif et optionnellement du tocopherol : Mélanger jusqu'à dissolution complète sous agitation cadre, de préférence 100 tr/min et 4000 tr/mn, à température inférieure à 25°C. L'aspect reste identique.
- Etape 10 : Préparation du premix solution acide citrique : Mélanger eau purifiée + acide citrique sous agitation pendant entre 3 et 8 minutes, de préférence 5 minutes. La solution est limpide, incolore.
- Etape 11 : Ajustement du pH entre 5.0 et 5.4 : Ajout si nécessaire d'une solution acide citrique 20% sous agitation, de préférence 150 tr/min, jusqu'à obtention pH entre 5.0 et 5.4. L'aspect est celui d'une crème blanche, brillante et homogène.
- Etape 12 : Qs Eau: Mélanger jusqu'à dispersion complète, sous agitation de préférence 150 tr/min à une température inférieure à 25°C.

L'aspect obtenu est une base crème blanche, brillante, aspect gel, homogène.

### Figures

[Fig 1] est une représentation schématique du flow chart de fabrication de la base de formulation selon l'invention
[Fig 2] est un graphique présentant les concentrations bactériennes des différentes souches en fonction des produits (S. aureus (en gris foncé), S. epidermidis (en gris clair)), après 8h d'incubation dans la co-culture, en lien avec l'exemple 1.
[Fig 3] est un graphique présentant les concentrations bactériennes des différentes souches en fonction des (S. aureus (en gris foncé), S. epidermidis (en gris clair)), après 24h d'incubation dans la co-culture, en lien avec l'exemple 1.
[Fig 4] est un graphique présentant les concentrations bactériennes des différentes souches en fonction des produits (S. aureus (en gris foncé), S. epidermidis (en gris clair)), après 8h d'incubation dans la co-culture, en lien avec l'exemple 3.
[Fig 5] est un graphique présentant les concentrations bactériennes des différentes souches en fonction des produits (S. aureus (en gris foncé), S. epidermidis (en gris clair)), après 24h d'incubation dans la co-culture, en lien avec l'exemple 3.
[Fig 6] est un graphique présentant les concentrations bactériennes des différentes souches en fonction des produits (S. aureus (en gris foncé), S. epidermidis (en gris clair)), après 24h d'incubation dans la co-culture, en lien avec l'exemple 3.
[Fig 7] est un graphique présentant les concentrations bactériennes des différentes souches en fonction des produits (S. aureus (en gris foncé), S. epidermidis (en gris clair)), après 24h d'incubation dans la co-culture, en lien avec l'exemple 3.
[Fig 8] est un graphique représentant le pourcentage de mortalité cellulaire en fonction de la concentration de la composition. La figure illustre la très faible mortalité des cellules SIRC après contact avec la composition, même à la concentration la plus élevée de 100%.

### Définitions

Au sens de la présente invention, l'expression « base de formulation cosmétique ou pharmaceutique » désigne une composition galénique qui constitue un véhicule. Elle se distingue d'une composition cosmétique ou pharmaceutique finale en ce qu'elle est structurellement dépourvue d'un principe actif d'indication. Un "principe actif d'indication" est entendu comme un principe actif (ou « actif », ou « actif cosmétique » ou « actif pharmaceutique ») dont la présence est justifiée par l'obtention d'un effet thérapeutique ou cosmétique principal et ciblé, tel qu'un effet anti-acnéique, anti-âge ou anti-inflammatoire. La base de formulation selon l'invention contient des excipients qui peuvent avoir des fonctions secondaires bénéfiques pour la peau (telles que l'hydratation ou la conservation), mais elle est conçue comme une plateforme stable, compatible et bien tolérée, apte à l'incorporation ultérieure d'au moins un principe actif d'indication pour former une composition finale.

Au sens de la présente invention, une formulation cosmétique désigne une composition destinée à être appliquée sur les parties superficielles du corps humain, notamment la peau, les lèvres, les ongles, les cheveux ou les muqueuses externes, dans un but non thérapeutique, notamment pour nettoyer, parfumer, hydrater, protéger, maintenir en bon état, modifier l'aspect ou corriger les odeurs corporelles. Une telle formulation peut se présenter sous différentes formes galéniques, telles que des crèmes, lotions, gels, laits, baumes, sprays, notamment buccaux ou nasaux, aérosol, mousses, patchs ou encore sérums. Elle peut contenir un ou plusieurs principe actif d'indication cosmétiques, tels que des agents hydratants, apaisants, séborégulateurs, anti-âge, dépigmentants, exfoliants, ou encore des filtres solaires. Elle peut également inclure des agents auxiliaires, tels que des agents de texture (gélifiants, épaississants), des émulsionnants, des conservateurs, des parfums, des colorants, ou des agents filmogènes, dans le respect de la réglementation applicable aux produits cosmétiques. De telles formulations peuvent être conditionnées en pot, flacon, tube, ou tout autre conditionnement qui conviendrait.

Par « non-thérapeutique » est entendu au sens de l'invention, une application cosmétique n'ayant pas vocation à traiter un patient. Une composition cosmétique n'agit pas comme un médicament pour le traitement de diverses maladies telles que l'acné, la xérose, le psoriasis ou encore l'eczéma, mais permet d'améliorer l'aspect visuel superficiel de la peau, chez un sujet sain.

Au sens de la présente invention, une formulation pharmaceutique désigne une composition contenant au moins une substance active destinée à prévenir, diagnostiquer, traiter ou soulager une pathologie ou un dysfonctionnement physiologique chez l'être humain ou l'animal. Une telle formulation comprend au moins un principe actif d'indication à visée thérapeutique et un ou plusieurs excipients pharmaceutiquement acceptables, permettant d'assurer la stabilité, la biodisponibilité, la tolérance et la conservation de la préparation. La formulation pharmaceutique peut se présenter sous des formes galéniques diverses, notamment : formes topiques (gel, crème, pommade, solution, lotion, sprays, notamment buccaux, aérosol), formes orales (comprimé, gélule, suspension), formes injectables (solution ou suspension injectable), formes mucoadhésives (patch, ovule, collyre, etc.), formes nasale (sprays nasaux,...), ou formes vaginales, selon la voie d'administration envisagée. De telles formulations peuvent être conditionnées en pot, flacon, tube, ou tout autre conditionnement qui conviendrait.

Au sens de la présente invention, « dispositif médical » désigne tout instrument, appareil, équipement, logiciel, implant, réactif ou autre article, utilisé seul ou en combinaison, chez l'être humain ou l'animal à des fins médicales, notamment pour le diagnostic, la prévention, la surveillance, le traitement ou l'atténuation d'une maladie ou d'une blessure, ou pour la compensation d'un handicap, l'étude ou la modification de l'anatomie ou d'un processus physiologique, ou la maîtrise de la conception. Dans le cadre de la présente invention, le dispositif médical est conçu pour contenir, transporter, délivrer, ou être mis en contact avec la base de formulation selon l'invention, ladite base étant destinée à être mélangée avec ou incorporer un ou plusieurs principes actifs. Le dispositif médical peut être, par exemple, un dispositif topique (pansement, patch), injectable (seringue préremplie), implantable (implant, cathéter), ophtalmique, nasal, auriculaire, gastro-intestinal, buccal, vaginal ou rectal. L'action principale du dispositif n'est pas obtenue par des moyens pharmacologiques, immunologiques ou métaboliques, bien que ceux-ci puissent intervenir à titre auxiliaire, conformément à la réglementation applicable en matière de dispositifs médicaux.

Au sens de la présente invention, l'expression « un ou plusieurs principe actif d'indication cosmétiques ou pharmaceutiques » désigne toute substance ou combinaison de substances ayant une activité biologique, physiologique ou fonctionnelle, destinée à produire un effet recherché sur ou dans l'organisme humain ou animal. Ces principe actif d'indication peuvent être de nature chimique, biotechnologique, végétale, minérale ou synthétique, et sont sélectionnés en fonction de l'effet souhaité. Ces principe actif d'indication peuvent être hydrosolubles ou liposolubles, incorporés sous forme libre ou vectorisée, et peuvent être ajoutés à la base de formulation avant, pendant ou après sa préparation, selon les propriétés physico-chimiques de l'actif et la stabilité de la formulation.

La base de formulation selon l'invention est conçue pour recevoir ultérieurement / être mélangée avec un ou plusieurs principe actif d'indication cosmétiques ou pharmaceutiques, choisis en fonction de l'effet recherché. Ces principes actifs d'indication peuvent être introduits par le formulateur ou l'utilisateur final, tout en maintenant la stabilité, la tolérance et l'efficacité du produit final.

Au sens de la présente invention, une émulsion gélifiée huile dans eau (H/E ou O/W) désigne une formulation biphasique comprenant : une phase continue aqueuse (eau ou solution aqueuse), dans laquelle est dispersée une phase discontinue huileuse (phase lipidique), la formulation étant gélifiée dans sa totalité ou partiellement au moyen d'un ou plusieurs agents gélifiants, hydrosolubles et/ou lipophiles. Cette structure associe les propriétés classiques d'une émulsion huile dans eau (texture légère, facilité de rinçage, bonne tolérance) à celles d'un gel (viscosité accrue, stabilité, application localisée, effet frais ou filmogène).

Au sens de la présente invention, l'expression « tolérance cutanée améliorée » désigne la capacité d'une formulation à minimiser les réactions indésirables au niveau de la peau lors de son application, en comparaison avec des formulations de référence ou similaires. Cette amélioration de la tolérance se manifeste notamment par une réduction de l'apparition de signes d'irritation, d'inflammation, de picotements, de rougeurs, de sécheresse excessive, de démangeaisons ou de sensations de brûlure, que ce soit immédiatement après l'application ou après usage répété. Ceci est démontré dans les exemples.

La base de formulation selon l'invention présente une tolérance cutanée améliorée, ce qui la rend particulièrement adaptée à un usage fréquent, prolongé ou sur des peaux sensibles, réactives, irritées ou atopiques, ou sur les muqueuses, nasale, buccale, vaginale, ou opthalmique..

Au sens de la présente invention, l'expression « activité bactéricide » désigne la capacité d'une composition à tuer activement des bactéries vivantes, et/ou inhiber leur croissance. Cette activité est démontrée dans les exemples. L'activité bactéricide visée dans le cadre de la présente invention cible en particulier :
- *Staphylococcus aureus,* une bactérie à Gram positif pathogène, souvent impliquée dans les infections cutanées (impétigo, folliculites, eczéma surin-fecté) et dans la dysbiose cutanée chez les peaux atopiques ou fragiles ;
- *Staphylococcus epidermidis,* également une bactérie à Gram positif, naturellement présente sur la peau mais pouvant devenir opportuniste, notamment dans les contextes de déséquilibre cutané, de port de dispositifs médicaux, ou de barrière altérée.

L'évaluation de l'activité bactéricide contre ces souches a été réalisée dans les exemples 1 et 3.

Au sens de la présente invention, l'expression « cosmétiquement ou pharmaceutiquement acceptable » qualifie un composé, un excipient, un véhicule, un support ou une formulation adapté à une application topique, muqueuse ou systémique sur le corps humain ou animal, et qui ne provoque pas, dans les conditions normales d'usage, d'effet indésirable significatif tel qu'une irritation, une sensibilisation, une toxicité ou une réaction allergique.

Au sens de la présente invention, un composé est dit cosmétiquement acceptable s'il est adapté à une utilisation non thérapeutique sur la peau, les cheveux, les ongles, les lèvres ou les muqueuses externes.

Au sens de la présente invention, un composé est dit pharmaceutiquement acceptable s'il est compatible avec une administration thérapeutique (topique, orale, injectable, etc.), et qu'il respecte les exigences de tolérance, sécurité, pureté, stabilité et efficacité définies par les pharmacopées ou les pratiques pharmaceutiques en vigueur.

La glycérine est utilisée comme agent humectant afin d'assurer une meilleure cohésion des cellules cutanées et former un film occlusif sur l'épiderme. Elle renforce ainsi le film hydrolipidique à la surface de la peau en apportant une protection vis-à-vis des agressions extérieures et limiter les risques d'irritation.

Les triglycérides d'acide caprylique et d'acide caprique sont des solvants huileux permettant de réaliser l'émulsion, composé d'un ester mixte composé d'acides gras capryliques et capriques fixés à une base de glycérine reconnu pour sa parfaite innocuité. Ces triglycérides apportent un côté doux pour la peau. Un tel produit est par exemple du Myritol 318^{®} de BASF.

Au sens de la présente invention, un « tensioactif » est un composé amphiphile, c'est-à-dire qu'il est constitué de deux parties distinctes : une partie hydrophobe (ou lipophile), qui présente une affinité pour les corps gras, et une partie hydrophile, qui présente une affinité pour l'eau. Cette structure double lui confère la capacité de se positionner aux interfaces entre deux phases non miscibles, comme l'huile et l'eau, permettant ainsi de réduire la tension interfaciale et de stabiliser des émulsions. La nature de la partie hydrophile, et en particulier sa charge électrique en milieu aqueux, détermine la classification du tensioactif. On distingue notamment :
- les tensioactifs anioniques, qui possèdent une tête polaire chargée négativement ;
- les tensioactifs cationiques, qui possèdent une tête polaire chargée positivement ;
- les tensioactifs amphotères (ou zwitterioniques), dont la charge varie en fonction du pH du milieu ; et
- les tensioactifs non-ioniques, qui sont dépourvus de charge nette sur leur tête polaire.

Un tensioactif non-ionique est un composé amphiphile dépourvu de charge électrique sur sa tête polaire, contrairement aux tensioactifs anioniques, cationiques ou amphotères. Il est constitué d'une partie hydrophobe, généralement une chaîne d'acide gras ou un groupement alkyle, et d'une partie hydrophile neutre, souvent à base de groupements polyéthylène glycol (PEG), alcools, sucres ou autres fonctions polaires non chargées.

Le lauryl glucoside, mélangé avec du myristyl glucoside et du polyglyceryl-6-laurate est un émulsionnant H/E non-ionique d'origine naturelle formulable à froid pour des émulsions sprayables, fluides à très fluides et des textures épaisses pour un toucher doux et léger. Un tel produit est par exemple du FLUIDIFEEL^{™} EASY^{®} de Seppic.

Les phospholipides hydrogénés sont des phospholipides dans lesquels tout ou partie des doubles liaisons présentes dans les chaînes d'acides gras ont été saturées par hydrogénation. Cette modification chimique confère aux phospholipides une stabilité accrue vis-à-vis de l'oxydation ainsi qu'un point de fusion plus élevé, ce qui peut améliorer leurs propriétés physiques, notamment en termes de stabilité thermique et de comportement dans des systèmes colloïdaux.

Le mélange d'oléate de polyglycéryle-6 et d'olivate de potassium est un système tensioactif, associant un ester de polyglycérol (l'oléate de polyglycéryle-6) et un sel d'acide gras (l'olivate de potassium), généralement issu d'huile d'olive.

Le polyglyceryl-2-dipolyhydroxystearate est un émulsifiant non-ionique dérivé de polyglycérol et d'acide polyhydroxystéarique.

Le polyglyceryl-3-diisostearate est un tensioactif non-ionique lipophile obtenu par estérification de polyglycérol avec de l'acide isostéarique.

Le myristate de polyglycéryle-10 est un émulsifiant non-ionique obtenu par estérification de polyglycérol (composé de 10 unités glycérol) avec de l'acide myristique.

La surfactine sodique (ou sodium surfactin) est un biosurfactant anionique produit par fermentation bactérienne, notamment par *Bacillus subtilis.*

Le mélange d'alcool cétéarylique et de glucoside de cétéaryle associe un alcool gras (alcool cétéarylique) à un tensioactif non-ionique doux (glucoside de cétéaryle).

Les extraits de graines de soja sont des ingrédients naturels riches en composés bioactifs tels que les isoflavones, les acides gras essentiels et les protéines.

La lécithine est un mélange naturel de phospholipides extrait principalement du soja ou du jaune d'œuf. Elle est utilisée comme agent tensioactif.

Le Polysorbate 80, ou Tween^{®} 80, est un tensioactif non-ionique synthétique obtenu par estérification de sorbitol et d'acide oléique, suivi d'une éthoxyla-tion. Il est utilisé comme solubilisant, dispersant ou stabilisant dans des préparations aqueuses contenant des huiles.

L'Oléate de sorbitan ou Span^{®} 80 est un tensioactif non-ionique lipophile résultant de l'estérification du sorbitan (dérivé du sorbitol) avec de l'acide oléique.

Un gélifiant est un agent structurant capable de former un réseau tridimensionnel au sein d'un liquide, conduisant à la formation d'un gel. Ce réseau peut résulter de liaisons physiques (interactions hydrophobes, liaisons hydrogène, associations micellaires) ou chimiques (liaisons covalentes, réticulation). Les gélifiants peuvent être d'origine naturelle (par exemple, gélatine, agar, pectine, alginates, gommes végétales) ou synthétique (par exemple, carbomères, polya-crylates).

La gomme de xanthane est un polysaccharide extracellulaire produit par fermentation bactérienne (notamment par Xanthomonas campestris). Elle est utilisée comme agent gélifiant et épaississant, même à faible concentration, et présente une bonne stabilité sur une large gamme de pH et de températures.

La cellulose microcristalline est un polymère naturel obtenu par hydrolyse acide partielle de la cellulose. Elle forme des gels pseudoplastiques, utilisés pour épaissir et stabiliser des émulsions ou suspensions.

L'agar est un mélange de polysaccharides (principalement agarose et agaropec-tine) extrait d'algues rouges. Il forme des gels thermoréversibles rigides en milieu aqueux à faible concentration.

Les carraghénanes sont des polysaccharides sulfatés extraits d'algues rouges du genre *Chondrus* ou *Gigartina.* Selon leur type (kappa, iota, lambda), ils forment des gels plus ou moins rigides ou élastiques.

Les pectines sont des polysaccharides riches en acide galacturonique, extraits de fruits (notamment agrumes ou pommes). Elles gélifient en présence de sucre et d'ions calcium, et sont utilisées comme agents de texture dans des produits alimentaires ou cosmétiques.

La gomme guar est un galactomannane extrait de la graine de guar *(Cyamopsis tetragonoloba*). Elle est utilisée comme gélifiant, en raison de sa capacité à former des solutions visqueuses en milieu aqueux, même à température ambiante.

La gomme de caroube (ou farine de graines de caroube) est un galactomannane extrait des graines du caroubier (*Ceratonia siliqua*). Elle forme des gels.

La gomme arabique est une résine naturelle séchée issue de la sève d'Acacia senegal ou *Acacia seyal.* Elle forme des gels très souples et est utilisée comme gélifiant.

La gélatine est un biopolymère obtenu par hydrolyse partielle du collagène animal. Elle forme des gels thermoréversibles dans l'eau à température ambiante, avec une texture souple.

Le collagène est une protéine fibreuse structurale présente dans les tissus conjonctifs. Sous forme native ou hydrolysée, il contribue à la formation de gels viscoélastiques.

La cellulose, de préférence ses dérivés comme la carboxyméthylcellulose ou l'hydroxypropylméthylcellulose, sont utilisés comme gélifiants grâce à leur capacité à former des réseaux hydratés visqueux.

Les amidons sont des polysaccharides naturels extraits de céréales ou tubercules (maïs, pomme de terre, riz). Ils peuvent former des gels plus ou moins rigides après cuisson et refroidissement.

Le chitosan est un polysaccharide cationique dérivé de la chitine, extraite principalement des carapaces de crustacés. En tant que gélifiant, il forme des gels en milieu acide grâce à ses interactions électrostatiques.

Les alginates sont des polysaccharides naturels extraits d'algues brunes, composés d'acide mannuronique (M) et d'acide guluronique (G). En tant que gélifiants, ils forment des gels en présence d'ions divalents, comme le calcium (Ca²⁺), par un processus de réticulation ionique. Les argiles (telles que la bentonite ou la montmorillonite) sont des silicates naturels en feuillets capables de former des gels colloïdaux dans l'eau.

La gomme sclérotique est un gélifiant polysaccharidique 100% naturel obtenu par fermentation de Sclerotium rolfsii en milieu enrichi en glucose. Un tel produit est par exemple de l'Amigum^{®} de Croda.

Le mélange de cellulose microcrystalline et de gomme de xanthane est un stabilisant/ gélifiant naturel qui bénéficie de l'effet synergique de ses composants pour conférer douceur et stabilité. Un tel produit est par exemple du Vivapur CS 032 XV^{®}.

Le tocopherol est un anti-oxydant naturel obtenu à partir d'huile végétale. Il protège les formules contre l'oxydation en bloquant les radicaux libres et permet de prolonger la stabilité des formules.

Le pentylène glycol d'origine naturelle possède une action « conservatrice » car il limite le développement des microbes et des bactéries et il est également reconnu pour ses propriétés humectantes, ce qui le rend particulièrement intéressant pour les peaux sèches et déshydratées. Un exemple de produit comprenant du pentylène glycol et pouvant être utilisé pour confectionner l'invention est Hydrolite^{®} 5 green (Symrise).

Le sorbate de potassium est un sel d'acides organiques existant à l'état naturel dans de nombreux végétaux. C'est un acide organique qui a une activité antimicrobienne, il est donc important d'avoir un pH acide (4.5 - 5.5) pour une bonne efficacité de conservation.

La solution d'acide citrique est ajoutée en fin de conception afin d'ajuster le pH entre 5.0 et 5.5 qui correspond au pH naturel de la peau.

Des exemples de principes actifs d'indication pouvant être utilisés avec les bases de formulation selon l'invention sont les suivants :
- ECOSKIN ^{®} (Solabia). Complexe pré/postbiotique obtenu par fermentation. Il est composé de α-glucooligosaccharides (GOS) obtenus par synthèse enzymatique à partir de substrats végétaux, de jus de plante 100% pur riche en β-fructooligosaccharides (FOS) obtenu par pressage à froid de tubercules de yacon (Polymnia sonchifolia), et de bactéries probiotiques lactobacillus (L. casei, L. acidophilus) inactivées. Renforce la barrière microbienne cutanée : Équilibre le paysage microbien de la peau, agissant comme substrat pour les bactéries commensales et renforçant les défenses naturelles de la peau. Régénère les peaux sèches : Lisse la surface cutanée, apportant confort et éclat. Rééquilibre les peaux grasses.
- BASHYAL ^{®} (Givaudan) est un acide hyaluronique hydrolysé de poids moléculaire moyen qui permet de renforcer les défenses naturelles de la peau, stimuler la libération de bêtadéfensines par les kératinocytes jusqu'à 600%, et activer la réparation cellulaire de la peau.
- SERENIBIOME ^{®} (Solabia). Serenibiome^{®} est un ingrédient actif innovant développé par le groupe Solabia, spécialement conçu pour les peaux sensibles et à tendance atopique. Il est composé de flocculosine et de propanediol. La Flocculosine est obtenue par biofermentation du champignon Pseu-dozyma flocculosa, cet ingrédient est inspiré par la nature et le concept de holobionte. Serenibiome^{®} inhibe sélectivement la croissance de *Staphylococcus aureus,* une bactérie opportuniste prévalente sur les peaux à tendance atopique, sans affecter la population de *Staphylococcus epidermidis.* Il permet de réduire l'inflammation neurogène : Diminue les marqueurs pro-inflammatoires impliqués dans la nociception et améliore la synthèse des molécules de bien-être, apaisant ainsi les sensations de démangeaison. Enfin il permet de réparer la barrière cutanée : Accélère le processus de ré-épithélialisation, favorisant la régénération et la réparation de la peau.

Dans le contexte de la présente invention, l'expression "un ou plusieurs des éléments A, B et C" dans une expression divulguant plusieurs caractéristiques/possibilités possibles, où chacun des éléments A, B et C représente des caractéristiques/possibilités spécifiques, doit être comprise comme divulguant explicitement soit A, soit B, soit C, soit A et B, soit A et C, soit B et C, soit A et B et C. À cet égard, il convient de noter que la personne du métier est capable de transposer l'expression abstraite susmentionnée à un exemple concret.

Dans la description et dans les exemples suivants, sauf indication contraire, les pourcentages sont des pourcentages en poids et les plages de valeurs libellées sous la forme « entre ... et ... » incluent les bornes inférieure et supérieure précisées. Les exemples ci-après sont présentés à titre illustratif et non limitatif du domaine de l'invention.

### Exemples

### Exemple 1 : Etude de l'activité bactéricide de la base de formulation selon l'invention.

### I - Introduction.

L'objectif de cet exemple est d'étudier l'activité bactéricide, bactériostatique ou au contraire « nourricier » de la multiplication bactérienne, de produits placés dans le cadre d'une co-culture de deux bactéries sauvages issues de la joue d'individus sains (i.e *Staphylococcus aureus* et *Staphylococcus epidermidis).*

### II - Matériel et méthode : Analyse de l'activité de produits dans le cadre d'une co-culture de deux bactéries.

Les souches suivantes ont été sélectionnées : *Staphylococcus aureus* (Sa.) & *Staphylococcus epidermidis* (Se.)
Les conditions de cultures adaptées à la bonne croissance des deux souches ensemble ont été établies au préalable. En conditions aérobie et dans un même milieu de culture, les deux souches coexistent de manière équilibrée. Les produits ont été préparés selon les recommandations du client aux concentrations présentées dans le *Tableau 3,* ci-dessous.

**[Table 3]**

| **Référence échantillon** | **Concentration** |
|---|---|
| **Base selon l'invention, sans conservateurs (Emulpro placebo sans conservateurs dans les figures)** | **100%** |
| **Base selon l'invention sans conservateur, avec 4% de Serenibiome^{®} (Emulpro 4% Serenibiome^{®} sans conservateurs dans les figures)** | **100%** |
| **Base selon l'invention avec conservateur, avec 4% de Serenibiome ^{®} (Emulpro 4% Serenibiome ^{®}dans les figures)** | **100%** |
| **Serenibiome ^{®}** | **4%** |

La dénomination « Emulpro » désigne la composition présentée dans le tableau 1 (Table 1), à savoir :

| **Composés** | **Fonction** | **Base selon l'invention (en % m/m)** |
|---|---|---|
| **Eau purifiée** | **Solvant** | **Qs 100** |
| **Glycérine** | **Agent humectant** | **5.00** |
| **Triglycéride d'acide caprylique et d'acide caprique;** | **Solvant huileux** | **20.00** |
| **Lauryl glucoside, myristyl glucoside et polyglyceryl-6-laurate** | **Emulsionnant** | **0.50** |
| **Gomme sclérotique** | **Agent gélifiant** | **1.00** |
| **Tocopherol** | **Anti-oxydant** | **0.30** |
| **Pentylène glycol** | **Conservateur** | **5.00** |
| **Sorbate de potassium** | **Conservateur** | **0.125** |
| **Acide citrique** | **Ajusteur de pH** | **Qs pH 5.0-5.5** |
| | **Total** | **100** |

L'indication « Sans conservateur » désigne que le pentylène glycol et le sorbate de potassium ne sont pas présents dans la formule dite « EMULPRO ». L'indication « Avec conservateur » désigne que le pentylène glycol et le sorbate de potassium sont présents dans la formule dite « EMULPRO ».

Plusieurs témoins ont été réalisés :
- Un témoin correspondant au phénoxyéthanol 0,5 %: absence de croissance ;
- Un témoin correspondant au milieu de croissance minimal des souches (TSB 1/10, pH=5,2);
- Un témoin correspondant au milieu de croissance optimal des souches (TSB, pH=5,2);

Dans ces conditions, l'activité des produits a été étudiée de la manière suivante :
- Dépôt des différents échantillons en tube, dans le milieu minimal de culture, à la concentration choisie. L'échantillon Serenibiome^{®} étant dilué dans le tampon TSB 1/10 à pH 5,2.
- Contamination de chaque échantillon par le mélange des deux souches dont le ratio initial à t0 correspond à 90% de S. epidermidis et 10 % de S. aureus (concentration : 103 cfu/mL).
- Incubation des échantillons et témoins pendant 24 heures, à 32°C.
- Prélèvements d'un aliquot de culture à 8 et 24 heures.
- Dépôt des prélèvements sur des géloses spécifiques aux souches sélectionnées puis incubation 48h à 37°C en aérobie. Après incubation, le dénombrement est effectué pour chaque souche.
- Comparaison des résultats par rapport aux concentrations des populations à T0h, T8h et T24h en absence de produit. Pour chaque échantillon, l'analyse est réalisée en triplicat.

### III - Résultats de la co-culture.

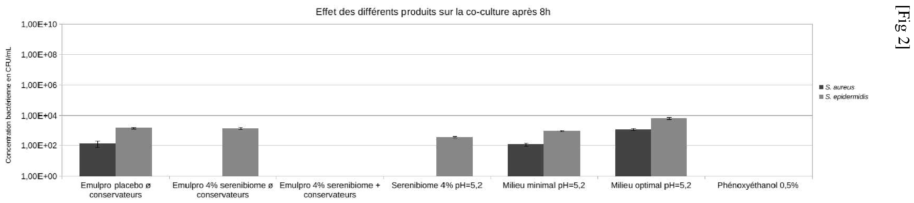

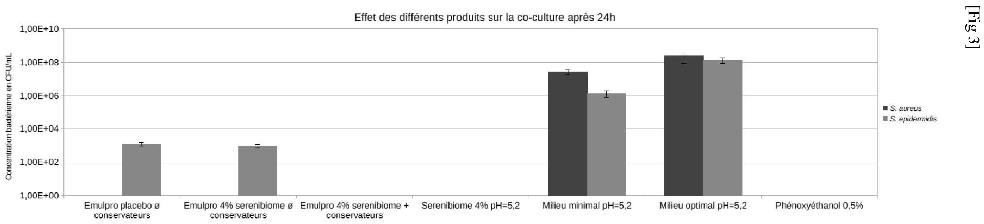

Les résultats sont présentés dans les figures 2 et 3.

Les résultats de l'analyse des 4 échantillons étudiés dans le cadre de cet exemple en co-culture après 24h d'analyse sont rassemblés dans les *Tableaux 4 et 5* ci-dessous.

**[Table 4] Bilan des réductions logarithmiques (RL) obtenues pour chaque souche par produit**

| **Produits** | **Réduction logarithmique' (RL) par espèce** | | | |
|---|---|---|---|---|
| | **8h** | | **24h** | |
| | ***S*. *aureus*** | ***S. epidermidis*** | ***S. aureus*** | ***S. epidermidis*** |
| **Base selon l'invention, sans conservateurs (Emulpro placebo sans conservateurs dans les figures)** | **0,1** | **0,2** | **-7,4** | **-3,1** |
| **Base selon l'invention sans conservateur, avec 4% de Serenibiome^{®} (Emulpro 4% Serenibiome^{®} sans conservateurs dans les figures)** | **-2,1** | **0,2** | **-7,4** | **-3,2** |
| **Base selon l'invention avec conservateur, avec 4% de Serenibiome^{®} (Emulpro 4% Serenibiome^{®} dans les figures)** | **-2,1** | **-3,0** | **-7,4** | **-6,1** |
| **Serenibiome^{®} 4%** | **-2,1** | **-0,4** | **-7,4** | **-6,1** |
| **Milieu minimal** | **0,0** | **0,0** | **0,0** | **0,0** |
| **Milieu optimal** | **1,0** | **0,9** | **1,0** | **2,0** |
| **Phénoxyéthanol 0,5%** | **-2,1** | **-3,0** | **-7,4** | **-6,1** |

Le tableau 4 décrit les valeurs des réductions logarithmiques obtenues pour chaque souche en fonction du produit testé à 8h et 24h.

Les réductions logarithmiques sont calculées de la manière suivante : RL_{espèce} x = (log UFC/mL du milieu minimum + bactéries en présence du produit testé) - (log UFC/mL du milieu minimum + bactéries en absence de produit)),

**[Table 5] Bilan des résultats obtenus à 8h et 24h (Pourcentage de chaque souche dans la co-culture en présence des différents produits et témoins).**

| **Produits** | **Réduction logarithmique (RL) par espèce** | | | |
|---|---|---|---|---|
| | **Sh** | | **24h** | |
| | ***S. aureus*** | ***S. epidermidis*** | ***S. aureus*** | ***S. epidermidis*** |
| **Base selon l'invention, sans conservateurs (Emulpro placebo sans conservateurs dans les figures)** | **8,3%** | **91,7%** | **0%** | **100%** |
| **Base selon l'invention sans conservateur, avec 4% de Serenibiome^{®} (Emulpro 4% Serenibiome ^{®} sans conservateurs dans les figures)** | **0%** | **100%** | **0%** | **100%** |
| **Base selon l'invention avec conservateur, avec 4% de Serenibiome^{®} (Emulpro 4% Se-rembiome^{®} dans les figures)** | **0%** | **0%** | **0%** | **0%** |
| **Serenibiome^{®} 4%** | **0%** | **100%** | **0%** | **0%** |
| **Milieu minimal** | **11,4%** | **88,6%** | **95,2%** | **4,8%** |
| **Milieu optimal** | **15,1%** | **84,9%** | **63,8%** | **36,2%** |
| **Phénoxyéthanol 0,5%** | **0%** | **0%** | **0%** | **0%** |

### IV - Conclusions

Les composés ont été testés sur le modèle de co-culture *S. aureus*/*S. epidermidis.* Les effets de ces composés sur le modèle de co-culture sont résumés dans le Tableau 6 ci-dessous.

**[Table 6] Interprétation de l'effet des produits étudiés dans le modèle de co-culture à 8h et 24h.**

| **Produits** | **Effet** |
|---|---|
| **Base selon l'invention, sans conservateurs (Emulpro placebo sans conservateurs dans les figures)** | **A 8h, pas d'effet sur les deux souches. A 24h, effet antibactérien total sur *S. aureus* et diminution de 3 log de la population de S. *epidermidis* (par rapport au milieu minimal).** |
| **Base selon l'invention sans conservateur, avec 4% de Serenibiome^{®} (Emulpro 4% Serenibiome^{®} sans conservateurs dans les figures)** | **A 8h et 24h, effet antibactérien total sur S. aureus. Pas d'effet sur S. *epidermidis à* 8h et diminution de 3 log à 24h (par rapport au milieu minimal).** |
| **Base selon l'invention avec conservateur, avec 4% de Serenibiome^{®} (Emulpro 4% Serenibiome^{®} dans les figures)** | **Effet antibactérien sur les deux souches (conservateurs).** |
| **Serenibiome^{®} 4%** | **A 8h, effet antibactérien sur S. aureus et à 24h, effet antibactérien sur les deux souches, à pH=5,2.** |

En conclusion,
- L'effet anti-bactérien du Serenibiome^{®} à 4% est observé, à pH=5,2, dès 8h sur la souche S. *aureus* et sur les deux souches à 24h. Comme lors de l'analyse précédente à 2%, l'effet anti-bactérien du Serenibiome^{®} est observé avec une cinétique plus rapide sur S. *aureus* par rapport à S. *epidermidis.*
- L'effet anti-bactérien du Serenibiome^{®} est également observé en formulation à 4%, sans conservateurs. L'effet anti-bactérien est total sur S. *aureus* à partir de 8h. Sur S. *epidermidis,* aucun effet n'est observé à 8h mais une diminution de 3 log est observée à 24h (par rapport au milieu minimal).
- Néanmoins, à 24 heures, il n'y a aucune différence entre l'effet de la formule selon l'invention (Emulpro) sans conservateurs, avec ou sans Serenibiome^{®} à 4 %.
- La formule selon l'invention (Emulpro) sans conservateurs démontre une efficacité sur *S*. *aureus.*

### Exemple 2 : Evaluation de l'effet de la base de formule selon l'invention sur l'expression de gènes versus Saforelle crème apaisante

Les effets des composant de la base de formule selon l'invention (EMULPRO Placebo lot 240513) et Saforelle^{®} Crème intime apaisante ont été évalués dans un modèle d'épiderme humain reconstruit (RHE) dans des conditions basales ou irradiées aux UV en évaluant la libération d'IL-8 et le profil d'expression des gènes à l'aide d'une technologie RT-qPCR. L'ARNm extrait a été analysé à l'aide d'une puce à PCR ciblant 128 gènes (dont 3 gènes de ménage) sélectionnés pour leur importance dans la physiologie des kératinocytes, le vieillissement cutané, la fonction barrière, l'inflammation ou le stress oxydatif.

### Matériels et méthodes :

### 2.1. Modèle biologique

- Épiderme : Épiderme humain reconstruit QIMA Bioalternatives (RHE), âgé de 10 jours :
- Lot n° 01015-737, pour l'essai préliminaire de cytotoxicité
- Lot n° 01015-743, pour les essais principaux.
- Conditions de culture : 37°C, 5% CO2
- Milieu d'essai : Milieu de maintenance QIMA Bioalternatives

### 2.2. Composés à tester

- Base de formule selon l'invention (EMULPRO Placebo lot 240513 ou EMULPRO PSO) (Table 1)

| **Composés** | **Fonction** | **Base selon l'invention** (en **% m/m)** |
|---|---|---|
| **Eau purifiée** | **Solvant** | **Qs 100** |
| **Glycérine** | **Agent humectant** | **5.00** |
| **Triglycéride d'acide caprylique et d'acide caprique;** | **Solvant huileux** | **20.00** |
| **Lauryl glucoside, myristyl glucoside et polyglyceryl-6-laurate** | **Emulsionnant** | **0.50** |
| **Gomme sclérotique** | **Agent gélifiant** | **1.00** |
| **Tocopherol** | **Anti-oxydant** | **0.30** |
| **Pentylène glycol** | **Conservateur** | **5.00** |
| **Sorbate de potassium** | **Conservateur** | **0.125** |
| **Acide citrique** | **Ajusteur de pH** | **Qs pH 5.0-5.5** |
| | **Total** | **100** |

Le terme PLACEBO ou PSO désigne qu'aucun principe actif cosmétique ou pharmaceutique n'est ajouté à la formule EMULPRO.
- Saforelle^{®} Crème intime apaisante

L'application est topique, et est réalisée sur 8 mg/cm² pour l'état basal et 5 et 8 mg/cm² pour la condition irradiée aux UV.

### 2.3. Essai préliminaire de cytotoxicité

- Epidermidis : RHE dans le milieu d'essai
- Durée d'incubation : 48 + 24 heures
- Paramètres d'évaluation : Essai de réduction du MTT

Après traitement, les RHE ont été incubés avec du MTT (sel de tétrazolium) réduit en cristaux de formazan bleu par la succinate déshydrogénase (enzyme mi-tochondriale). Cette transformation est proportionnelle à l'activité enzymatique. Après dissociation des cellules et solubilisation des cristaux de formazan à l'aide d'isopropanol/HCl, la densité optique (DO) des extraits à 540 nm, proportionnelle au nombre de cellules vivantes et à leur activité métabolique, a été enregistrée à l'aide d'un spectromètre (VERSAmax, Molecular Devices).

### 2.4. Culture et traitement

Des RHE âgés de dix jours ont été placés dans un milieu d'essai topiquement traité ou non (contrôle) avec les composés et incubés pendant 48 heures.

Pour les conditions d'irradiation : Les composés ont été retirés, les RHE ont ensuite été irradiés (en l'absence des composés testés) avec des UVB (+UVA) - 850 mJ/cm² (+ 6,4 J/cm²) à l'aide d'un simulateur solaire SOL500 équipé d'un filtre H2 (Dr. Hönle, AG). Après l'irradiation, les RHE ont été traités à nouveau ou non (contrôle) avec les composés testés pendant 24 heures d'incubation.

Pour les conditions de base : Les composés ont été retirés et les RHE ont été maintenus dans l'obscurité pendant la durée de l'irradiation. Les RHE ont ensuite été traités à nouveau ou non (contrôle) avec les composés testés pendant 24 heures d'incubation.

Parallèlement, un contrôle non irradié a été effectué et conservé dans l'obscurité pendant la durée de l'irradiation.

Toutes les conditions expérimentales ont été réalisées en n=3.

À la fin de l'incubation, des quantifications de la libération d'IL-8 ont été effectuées sur les sous-natants de culture. Les RHE ont été rincés avec du PBS et coupés en 2 parties :
- Une moitié pour l'analyse de l'expression génique,
- Une moitié a été conservée et congelée dans un milieu de congélation des tissus pour d'éventuelles analyses histologiques.

### 2.5. Test d'immuno-absorption enzymatique (ELISA)

L'IL-8 libéré dans les surnageants de culture a été mesuré à l'aide d'un kit ELISA spécifique (par exemple DuoSet CXCL8/IL-8), conformément aux instructions du fournisseur.

### 2.6. Analyse de l'expression différentielle

L'expression des marqueurs a été analysée par la méthode RT-qPCR sur l'ARN total extrait de RHE de chaque condition expérimentale (avant l'extraction de l'ARN, les réplicats de la même condition expérimentale ont été regroupés).

L'analyse des transcrits a été réalisée en n=2 à l'aide d'une matrice PCR ciblant 128 gènes (125 gènes + 3 gènes de ménage) sélectionnés pour leur importance dans la physiologie des kératinocytes, le vieillissement de la peau, la fonction barrière, l'inflammation ou le stress oxydatif.

### 2.6.1. Extraction de l'ARN et transcription inverse

Les échantillons ont été homogénéisés mécaniquement à l'aide de l'homogénéisateur d'échantillons Precellys^{®} Evolution et Cryolys^{®} (Bertin Instrument) et l'ARN total a été extrait à l'aide du Tripure Isolation Reagent^{®} selon les instructions du fournisseur.

La qualité de l'ARN a été évaluée par électrophorèse capillaire (4200 TapeSta-tion System ou Bioanalyzer 2100, Agilent technologies). La quantité d'ARN a été évaluée à l'aide d'un spectrophotomètre (Synergy H1, BioTek Instruments). Les contrôles de qualité de l'ARN sont présentés en annexe (figures 1 et 2).

L'ADN complémentaire (ADNc) a été synthétisé par transcription inverse de l'ARN total en présence d'oligo(dT) et de « Transcriptor Reverse Transcriptase » (Roche). Les quantités d'ADNc ont ensuite été ajustées avant l'étape de PCR.

### 2.6.2. PCR quantitative

Les PCR (Polymerase Chain Reaction) ont été réalisées à l'aide du système « LightCycler^{®} » (Roche Molecular System Inc.) selon les instructions du fournisseur.

Le mélange réactionnel (10 µl final) a été préparé comme suit :
- 2,5 µl d'ADNc,
- amorces (directes et inverses),
- mélange de réactifs (Ozyme) contenant de l'ADN polymérase taq, du SYBR Green I et du MgCl2.

### 2.7. Gestion des données

### 2.7.1. Essai préliminaire de cytotoxicité et libération d'IL-8

Les comparaisons entre groupes ont été effectuées à l'aide d'un test t de Student non apparié. L'analyse statistique peut être interprétée si n≥5, cependant pour n<5 les valeurs statistiques sont données à titre indicatif.

Formules utilisées dans ce rapport :
Erreur standard de la moyenne : sem = Sd/√n

L'erreur standard de la moyenne (sem) est une mesure de la distance probable entre la moyenne de l'échantillon et la moyenne réelle de la population. Elle est calculée en divisant l'écart-type par la racine carrée de la taille de l'échantillon.
Pourcentage de protection (%): [Math 1] $\frac{Moyenne du contr ô {le}_{\left(+UV\right)} - {Valeur}_{\left(+UV\right)}}{Moyenne du {contrôle}_{\left(+UV\right)} - Moyenne du {contrôle}_{\left(-U\right)}} \times 100$
Pourcentage de viabilité (%): [Math 2] $\left({OD}_{sample}/{OD}_{control}\right) \times 100$
Pourcentage de stimulation (%): [Math 3] $\left\{\frac{Valeur}{Moyenne du contrôle}\times 100\right\} - 100$

### 2.7.2. Gestion des données de la PCR quantitative

L'incorporation de la fluorescence dans l'ADN amplifié a été mesurée en continu pendant les cycles PCR.

Il en résulte un diagramme « intensité de fluorescence » en fonction du « cycle PCR » permettant l'évaluation d'une valeur d'expression relative (ER) pour chaque marqueur.

La valeur sélectionnée pour les calculs d'ER est le « point de sortie » (Ct) de la courbe de fluorescence. Pour un marqueur donné, la valeur la plus élevée est le nombre de cycles ; la plus faible est la quantité d'ARNm.

La valeur de l'ER a été calculée à l'aide de la formule suivante (1/2^{nombre de cycles})x 10⁶.

La matrice PCR utilisée dans la présente étude comprenait initialement 3 gènes de référence (GAPDH, RPS28 et ACTB).

Ces gènes sont utilisés pour la normalisation des données car leur expression est constitutive et théoriquement stable.

Cependant, comme l'expression de RPS28 et ACTB était significativement modulée par la base de formulation selon l'invention (EMULPRO PSO) et Saforelle^{®} Crème intime apaisante à la plus forte concentration testée, RSP28 et ACTB ont été exclus des calculs de normalisation des données et l'expression relative moyenne des gènes de référence a été calculée en utilisant uniquement la GAPDH pour toutes les conditions de test. Classification des effets (conditions « traitées » contre « contrôle non irradié » ou « contrôle »)

### Remarques concernant le protocole utilisé :

La méthode RT-qPCR est très sensible, mais cette sensibilité peut entraîner un niveau de variabilité. De plus, pour un marqueur déterminé, la quantité d'ARNm dans la cellule peut fortement varier en fonction du temps de traitement.

Ainsi, les résultats observés reflètent le profil d'expression au moment choisi. Pour une interprétation standardisée, QIMA Bioalternatives propose les valeurs suivantes :
Expression relative (% du contrôle) :
> 300% = Forte stimulation
> 200% et < 300% = Stimulation
> 150% et < 200% = Légère stimulation, à confirmer
> 50 % et < 65 % = Inhibition modérée, à confirmer
> 30 % et < 50 % = Inhibition
< 30 % = Forte inhibition

### 3. RÉSULTATS

### 3.1. Essai préliminaire de cytotoxicité

Les résultats sont présentés dans le tableau qui suit.

**[Table 7] Effet des composés EMULPRO PSO et Saforelle^{®} Crème intime apaisante sur la viabilité des RHE après 48 + 24 heures d'incubation**

| **Composés testés** | **Concentration** | **Absor-bance** | **Moyenne** | **Sem** | **% via bi-lité** | **se m** |
|---|---|---|---|---|---|---|
| | **(Application topique)** | **(OD540nm )** | **(OD540nm )** | **(OD540nm )** | | **(%)** |
| **Control** | **-** | **0.775** | **0.757** | **0.019** | **10 0** | **2** |
| | | **0.738** | | | | |
| **EMULPRO PSO** | **1.5 µl/RHE (2 mg/cm²)** | **0.626** | **0.693** | **0.067** | **92** | **9** |
| | | **0.759** | | | | |
| | **3.8 µl/RHE (5 mg/cm²)** | **0.626** | **0.672** | **0.046** | **89** | **6** |
| | | **0.717** | | | | |
| | **6 µl/RHE (8 mg/cm²)** | **0.660** | **0.686** | **0.026** | **91** | **3** |
| | | **0.712** | | | | |
| **Saforelle^{®} Crème intime apai- sante** | **1.5 µl/RHE (2 mg/cm²)** | **0.667** | **0.709** | **0.042** | **94** | **6** |
| | | **0.751** | | | | |
| | **3.8 µl/RHE (5 mg/cm²)** | **0.652** | **0.683** | **0.031** | **90** | **4** |
| | | **0.713** | | | | |
| | **6 µl/RHE (8 mg/cm²)** | **0.768** | **0.737** | **0.031** | **97** | **4** |
| | | **0.706** | | | | |

Les résultats de l'essai de réduction MTT ont permis de déterminer les concentrations à tester (voir paragraphe 2.2).

### 3.2. Effet sur la libération d'IL-8

Les résultats sont présentés dans les tableaux qui suivent.

Ci-dessous le tableau 8.

**[Table 8] Effet des composés EMULPRO PSO et Saforelle^{®} Crème intime apaisante sur la libération d'IL-8 par l'épiderme humain reconstruit en conditions basales.**

| **Composés testés** | **Concentrati on** | **Il-8** | **Moyenne** | **Sem** | **% contrôle** | **sem** | **P⁽¹⁾** |
|---|---|---|---|---|---|---|---|
| | **(Applicatio n topique)** | **(pg/ml))** | **(pg/ml)** | **(pg/m** | | **(%)** | |
| **Control** | **-** | **<31** | **<31** | **0** | **100** | **0** | **-** |
| | | **<31** | | | | | |
| | | **<31** | | | | | |
| **EMULPR O PSO** | **Pure (8 mg/cm²)** | **<31** | **<31** | **0** | **< 100** | **0** | **ns** |
| | | **<31** | | | | | |
| | | **<31** | | | | | |
| **Saforelle^{®} Crème intime apaisante** | **1.5 µl/RHE (2 mg/cm²)** | **<31** | **<31** | **0** | **< 100** | **0** | **ns** |
| | | **<31** | | | | | |
| | | **<31** | | | | | |

| | | | | | | | |
|---|---|---|---|---|---|---|---|
| *(1): Seuil de signification statistique* *ns : > 0,05, non significatif* * : *0,01 à 0,05, significatif* ** *: 0,001 à 0,01, Très significatif* *** : < *0,001, extrêmement significatif* *< ou > : Inférieur ou supérieur à la limite de détection* *< 31,250 ou > 2000 pg*/*ml pour les échantillons non dilués* < 93,75 *ou > 6000 pg*/*ml pour les échantillons dilués à 1*/*3* *En cas de valeurs < ou > aux limites de détection, les calculs suivants sont extrapolés.* | | | | | | | |

Ci-dessous le tableau 9.

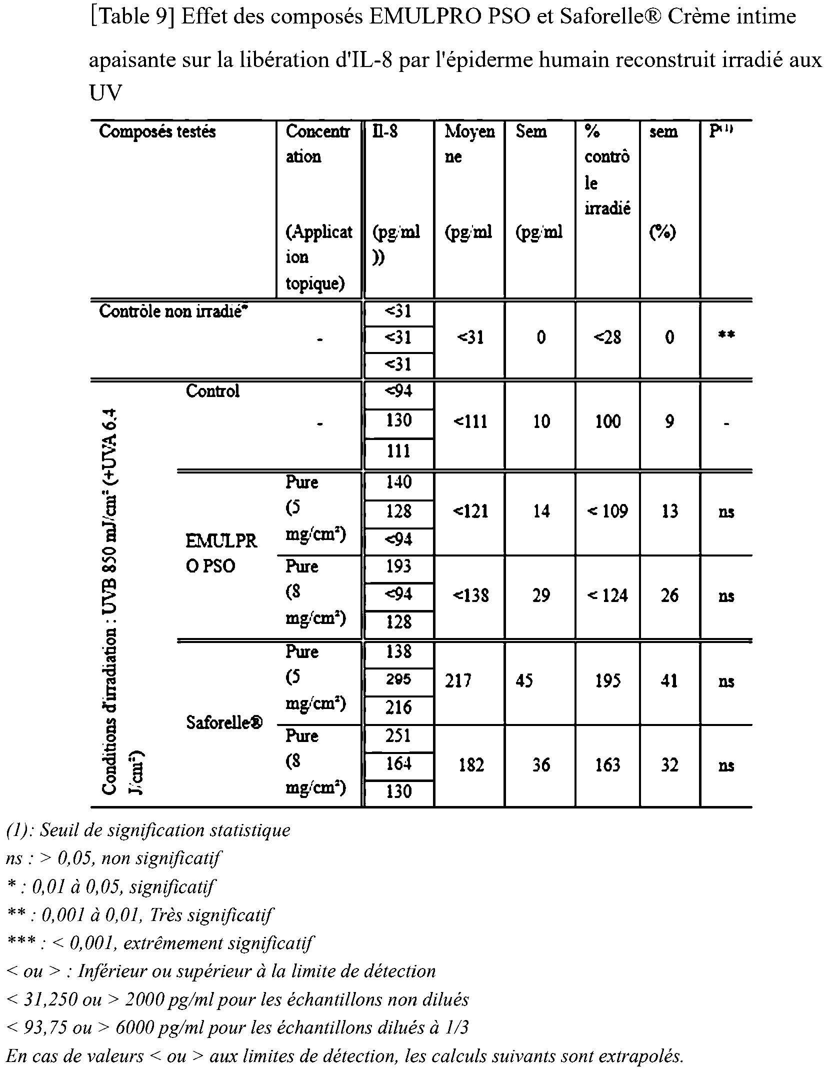

Dans ces conditions expérimentales, le contrôle non irradié n'a montré aucune libération détectable d'IL-8.

Le traitement de l'épithélium reconstruit avec les composés EMULPRO PSO et Saforelle^{®} Crème intime apaisante n'a montré aucune augmentation de la libération d'IL-8 dans les conditions de base.

L'irradiation de l'épiderme reconstruit a induit une augmentation significative de la libération d'IL-8 (~111 pg/ml).

Le traitement de l'épiderme reconstruit avec les composés EMULPRO PSO n'a pas clairement modulé la libération d'IL-8, tandis que Saforelle^{®} Crème intime apaisante a surstimulé la libération d'IL-8 (jusqu'à 217 pg/ml) par l'épiderme reconstruit irradié aux UV.

### 3.3. Effet sur l'expression des gènes

Dans les conditions expérimentales de cette étude, l'irradiation UV du RHE a induit, comme prévu, une forte signature sur l'expression des gènes.

Une analyse macroscopique montre qu'EMULPRO PSO a modulé très peu de gènes en conditions basales ou irradiées par les UV par rapport à Saforelle^{®} Crème intime apaisante.

Dans des conditions basales, EMULPRO PSO a stimulé l'expression de FOS et inhibé l'expression de MMP9 et MMP1. Le gène FOS code pour une protéine qui fait partie du complexe de facteurs de transcription AP-1, qui régule la prolifération, la différenciation, la réponse au stress, l'inflammation et l'apoptose des kératinocytes. Ce gène est essentiel au maintien de l'intégrité de la peau, à la cicatrisation des plaies et à la modulation des réponses immunitaires. Parallèlement, les MMP-1 et MMP-9 sont des métalloprotéinases matricielles impliquées dans le remodelage de la matrice extracellulaire. La MMP-1 contribue principalement à la dégradation du collagène, tandis que la MMP-9 cible la gélatine et l'élastine, toutes deux cruciales pour la cicatrisation des plaies, la migration cellulaire et les processus de réparation de la peau.

Sous stimulation UV, la base de formulation selon l'invention (EMULPRO PSO) a modulé plusieurs gènes (qui dépassent 170 % du niveau de contrôle) et a spécifiquement régulé à la baisse le GJA1. Les résultats sont résumés dans le tableau ci-dessous :

**[Table 10]**

| Fonction | Gènes |
|---|---|
| Barrière cutanée | GJA1, LAMC2, ITGB4, EPPK1, ITGA2, SDC1 |
| Cicatrisation des plaies | TGFB1, MMP1, MMP3, MMP9 |
| Angiogenèse | VEGFA, VEGFC |
| Hydratation de la peau | AQP3 |
| Réponse au stress oxydatif et Défense antioxydante | SOD1 |
| Réponse inflammatoire | PTGS2 |

Parallèlement, en conditions basales, Saforelle^{®} Crème intime apaisante a modulé plusieurs gènes (ceux supérieurs à 170% et inférieurs à 50% du niveau de contrôle). Les résultats sont résumés dans le tableau ci-dessous :

**[Table 11]**

| Fonction | Gènes |
|---|---|
| Barrière cutanée | SPRR1A, SPRRIB, EPPK1, LCE3D |
| Défense antimicrobienne | RNASE7, DEFB4A |
| Réponse au stress oxydatif et Défense antioxydante | MT1G |
| Réponse inflammatoire | CXCL10 |
| Cicatrisation des plaies | MMP1, MMP3, MMP9 |
| Inhibition de l'apoptose | BIRC5 |
| Métabolisme des lipides | SMPD1 |

Parallèlement, sous stimulation UV, Saforelle^{®} Crème intime apaisante, testée à la plus forte concentration, a induit une forte inhibition de plusieurs gènes pouvant être interprétée comme un début de cytotoxicité bien qu'aucune modification de la qualité de l'ARN n'ait été observée. Ces résultats sont cohérents avec la surstimulation IL-8 observée. Ces modulations ne seront pas discutées plus avant dans ce rapport.

Testée à la concentration la plus faible, sous stimulation UV, Saforelle^{®} Crème intime apaisante a modulé plusieurs gènes (ceux supérieurs à 170 et inférieurs à 50% du niveau de contrôle). Les résultats sont résumés dans le tableau ci-dessous :

**[Table 12]**

| Fonction | Gènes |
|---|---|
| Barrière cutanée | CRNN, LOR, SPRR1A, LAMC2, ITGB4, ITGB6, HSPG2, CD44 |
| Cicatrisation des plaies | TGFB1, SPARC, TNC, PLAU, HAS3, MMP3, MMP9 |
| Angiogenèse | VEGFC |
| Hydratation de la peau | AQP3 |
| Apoptose et prolifération cellulaire | FOS, BAX |
| Défense antimicrobienne | CAMP, RNASE7, S100A10, TLR2 |
| Réponse inflammatoire | IL8, TLNR2, PTGS2 |
| Réponse au stress oxydatif et défense antioxydante | SOD1, GSR, MT1G |
| Protéines de choc thermique (réponse au stress) | HSPA6, HSPA1A |

### 4. CONCLUSION

D'après cet exemple, la base de formulation selon l'invention (EMULPRO PSO) a démontré, dans des conditions basales, un effet minimal sur la libération et l'expression génétique de l'IL-8, ce qui le rend adapté aux peaux sensibles. En revanche, Saforelle^{®} Crème intime apaisante a montré une activité biologique étendue, améliorant la fonction de barrière cutanée, la cicatrisation des plaies et les défenses antimicrobiennes dans des conditions basales. Cependant, en cas de stress UV, Saforelle^{®} Crème intime apaisante a surstimulé la libération d'IL-8 et modulé un large éventail de gènes, ce qui indique un potentiel d'augmentation de l'inflammation et de la cytotoxicité à des concentrations plus élevées. Par conséquent, bien que les deux composés contribuent à maintenir la santé de la peau, la base de formulation selon l'invention (EMULPRO PSO) semble potentiellement plus approprié pour les peaux sensibles, tandis que Saforelle^{®} Crème intime apaisante offre des avantages substantiels en termes de barrière et de cicatrisation, mais présente une toxicité potentielle à fortes doses.

### Exemple 3 : Étude de l'action de la base de formulation selon l'invention (EMULPRO) et le Serenibiome^{®} sur le microbiote en co-culture

Cette étude a été réalisée pour vérifier l'activité antibactérienne de formule Emulpro avec et sans Serenibiome^{®} sur des souches bactérienne sauvages issus de volontaires sains dans le cadre d'une co-culture de deux bactéries sauvages issues de la joue d'individus sains (i.e *Staphylococcus aureus* et *Staphylococcus epidermidis).*

Plusieurs formules testées :
- Base de formulation selon l'invention (EMULPRO) avec 2 et 4% de Serenibiome^{®}
- Base de formulation selon l'invention (EMULPRO) sans conservateurs
- Base de formulation selon l'invention (EMULPRO) avec conservateurs
- Base de formulation selon l'invention (EMULPRO) sans conservateurs avec 2 et 4% de Serenibiome^{®}
- Serenibiome^{®} (matière active) à 2 et 4%

### I - Introduction.

L'objectif de cet exemple est d'étudier l'activité bactéricide, bactériostatique ou au contraire « nourricier » de la multiplication bactérienne, de produits placés dans le cadre d'une co-culture de deux bactéries sauvages issues de la joue d'individus sains (i.e *Staphylococcus aureus* et *Staphylococcus epidermidis).*

Pour cela, a été réalisé une adaptation en milieu liquide permettant d'augmenter le nombre d'analyses simultanées.

### II - Matériel et méthode : Analyse de l'activité de produits dans le cadre d'une co-culture de deux bactéries.

Les souches suivantes ont été sélectionnées *: Staphylococcus aureus* (Sa.) & *Staphylococcus epidermidis* (Se.)
Les conditions de cultures adaptées à la bonne croissance des deux souches ensemble ont été établies au préalable. En conditions aérobie et dans un même milieu de culture, les deux souches coexistent de manière équilibrée. Les produits ont été préparés selon les recommandations du client aux concentrations présentées dans le *Tableau 3,* ci-dessous.

**[Table 13]**

| **Référence échantillon** | **Concentration** |
|---|---|
| Base selon l'invention, sans conservateurs (Emulpro placebo sans conservateurs dans les figures) | 100% |
| Base selon l'invention, avec 2% de Serenibiome^{®} (Emulpro 2% Serenibiome dans les figures) | 100% |
| Base selon l'invention sans conservateur, avec 2% de Serenibiome^{®} (Emulpro 2% Serenibiome^{®} sans conservateurs dans les figures) | 100% |
| Base selon l'invention avec 4% de Serenibiome^{®} (Emulpro 4% Serenibiome^{®} dans les figures) | 100% |
| Serenibiome^{®} | 2% |

Plusieurs témoins ont été réalisés :
- Un témoin correspondant au phénoxyéthanol 0,5 %: absence de croissance ;
- Un témoin correspondant au milieu de croissance minimal des souches (TSB 1/10, pH=7);
- Un témoin correspondant au milieu de croissance optimal des souches (TSB, pH=7);

Dans ces conditions, l'activité des produits a été étudiée de la manière suivante :
- Dépôt des différents échantillons en tube, dans le milieu minimal de culture, à la concentration choisie.
- Contamination de chaque échantillon par le mélange des deux souches dont dont les proportions initiales permettent d'obtenir aux différents temps de prélèvement, un ratio 9:1 (90%:10%, S. aureus : S. epidermidis).- Incubation des échantillons et témoins pendant 24 heures, à 32°C.
- Prélèvements d'un aliquot de culture à 8 et 24 heures.
- Dépôt des prélèvements sur des géloses spécifiques aux souches sélectionnées puis incubation 48h à 37°C en aérobie. Après incubation, le dénombrement est effectué pour chaque souche.
- Comparaison des résultats par rapport aux concentrations des populations à T0h, T8h et T24h en absence de produit. Pour chaque échantillon, l'analyse est réalisée en triplicat.

### III - Résultats de la co-culture.

### III.1 - Premier essai - Protocole et méthodologie GLYcoDiag.

Concentrations à T0 : Sa 5.104 cfu/mL, Se 105 cfu/mL soit un ratio 1:2.

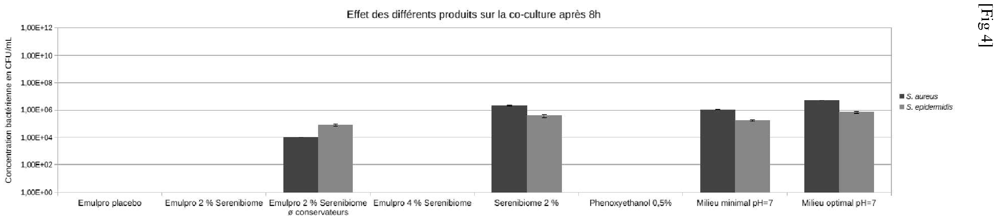

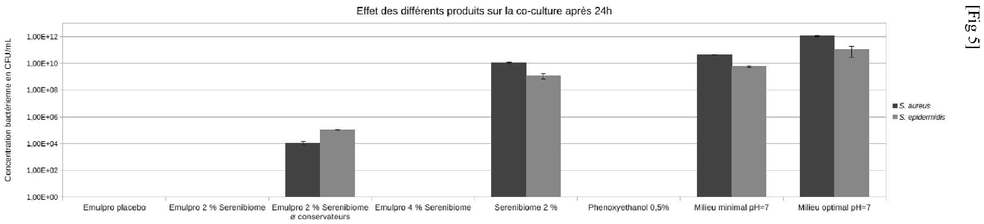

Les résultats sont présentés dans les figures 4 et 5.

Les résultats de l'analyse des 5 échantillons étudiés dans le cadre de ce projet en co-culture après 24h d'analyse sont rassemblés dans les Tableaux 14 et 15 ci-dessous.

**[Table 14] Bilan des réductions logarithmiques (RL) obtenues pour chaque souche par produit**

| **Produits** | **Réduction logarithmique¹ (RL) par espèce** | | | |
|---|---|---|---|---|
| | **8h** | | **24h** | |
| | **S. aureus** | **S. epidermidis** | **S. aureus** | **S. epidermidis** |
| **Base selon l'invention (Emulpro placebo dans les figures)** | **-6,0** | **-5,2** | **-10,7** | **-9,8** |
| **Base selon l'invention, avec 2% de Serenibiome^{®} (Emulpro 2% Serenibiome^{®} dans les figures)** | **-6,0** | **-5,2** | **-10,7** | **-9,8** |
| **Base selon l'invention sans conservateur, avec 2% de Serenibiome^{®} (Emulpro 2% Serenibiome^{®} sans conservateurs dans les figures)** | **-2,0** | **-0,3** | **-6,6** | **-4,7** |
| **Base selon l'invention avec 4% de Serenibiome^{®} (Emulpro 4% Serenibiome^{®} dans les figures)** | **-6,0** | **-5,2** | **-10,7** | **-9,8** |
| **Serenibiome^{®} 2%** | **0,3** | **0,3** | **-0,6** | **-0,7** |
| **Milieu minimal** | **0** | **0** | **0** | **0** |
| **Milieu optimal** | **0,7** | **0,6** | **1,4** | **1,3** |
| **Phénoxyéthanol 0,5%** | **-6,0** | **-5,2** | **-10,7** | **-9,8** |

Le tableau 14 décrit les valeurs des réductions logarithmiques obtenues pour chaque souche en fonction du produit testé à 8h et 24h.

Les réductions logarithmiques sont calculées de la manière suivante : RL_{espèce} x = (log UFC/mL du milieu minimum + bactéries en présence du produit testé) - (log UFC/mL du milieu minimum + bactéries en absence de produit)).

**[Table 15] Bilan des résultats obtenus à 8h et 24h (Pourcentage de chaque souche dans la co-culture en présence des différents produits et témoins).**

| **Produits** | **Réduction logarithmique¹ (RL) par espèce** | | | |
|---|---|---|---|---|
| | **8h** | | **24h** | |
| | ***S. aureus*** | ***S. epidermidis*** | ***S. aureus*** | ***S. epidermidis*** |
| **Base selon l'invention (Emulpro placebo dans les figures)** | **0%** | **0%** | **0%** | **0%** |
| **Base selon l'invention, avec 2% de Serenibiome^{®} (Emulpro 2% Serenibiome^{®} dans les figures)** | **0%** | **0%** | **0%** | **0%** |
| **Base selon l'invention sans conservateur, avec 2% de Serenibiome^{®} (Emulpro 2% Serenibiome^{®} sans conservateurs dans les figures)** | **10,7%** | **89,3%** | **9,1%** | **90,9%** |
| **Base selon l'invention avec 4% de Serenibiome^{®} (Emulpro 4% Serenibiome^{®} dans les figures)** | **0%** | **0%** | **0%** | **0%** |
| **Serenibiome^{®} 2%** | **84,9%** | **15,1%** | **90,3%** | **9,7%** |
| **Milieu minimal** | **86,3%** | **13,7%** | **88,5%** | **11,5%** |
| **Milieu optimal** | **88,3%** | **11,7%** | **91,0%** | **9,0%** |
| **Phénoxyéthanol 0,5%** | **0%** | **0%** | **0%** | **0%** |

Ces résultats permettent de mettre en évidence :
- L'effet antibactérien des conservateurs dans les formules suivantes (Emulpro = base selon l'invention) : Emulpro placebo, emulpro 2% Serenibiome et Emulpro 4% serenibiome^{®}.
- L'effet anti-bactérien de la formule Emulpro 2% Serenibiome^{®} sans conservateurs, en particulier sur la souche S. *aureus* à 8h, et sur les deux souches à 24h.
- L'absence d'effet du Serenibiome^{®} à 2%.

Ces premiers résultats ont montré un effet anti-S. *aureus* uniquement dans le cas de la formule Emulpro 2 % Serenibiome^{®} sans conservateurs.

Toutefois, une nouvelle étude a été réalisée en variant les conditions, et l'effet anti-bactérien du Serenibiome^{®} seul à 2 % sur la souche S. aureus n'ayant pas été observé. Ce nouvel essai est toujours réalisé selon la méthodologie GLYcoDiag, mais en adaptant ce protocole à celui décrit par Solabia. Les paramètres suivants ont été modifiés :
- Le ratio des souches microbiennes à t0 a été modifié, passant d'un ratio 1:2 à un ratio 1:9 *(S. aureus, S. epidermidis).*
- La concentration initiale des souches microbiennes passant de 105 cfu/mL à 103 cfu/mL.
- Le pH. En effet, pour suivre le protocole de Solabia, le pH du milieu de culture a été abaissé à 5.2. En parallèle, les témoins d'analyse ont également été étudiés à pH=7 (milieu minimum, milieu optimum et phénoxyéthanol).

### III.2. Second essai - Protocole Solabia et méthodologie GLYcoDiag.

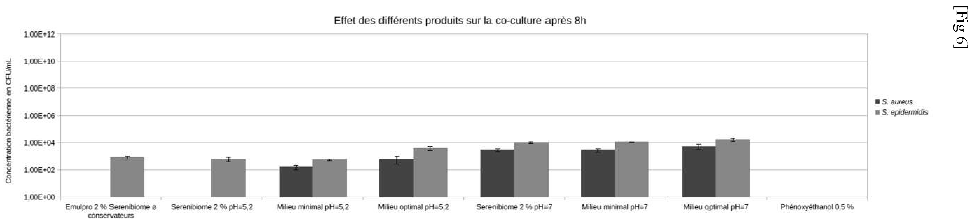

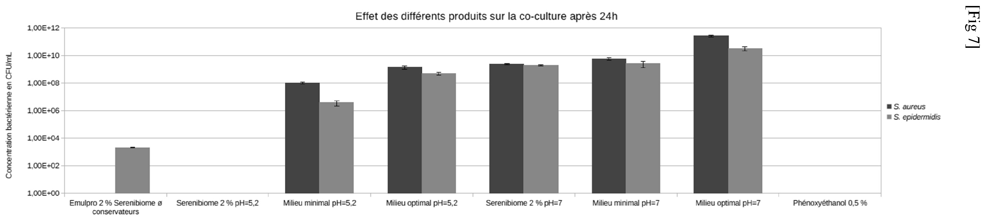

Les résultats sont présentés dans les figures 6 et 7.

Les résultats de l'analyse des 5 échantillons étudiés dans le cadre de ce projet en co-culture après 24h d'analyse sont rassemblés dans les Tableaux 16 et 17 ci-dessous.

**[Table 16] Bilan des réductions logarithmiques (RL) obtenues pour chaque souche par produit**

| **Produits** | **Réduction logarithmique¹ (RL) par espèce** | | | |
|---|---|---|---|---|
| | **8h** | | **24h** | |
| | ***S. aureus*** | ***S. epidermidis*** | ***S. aureus*** | ***S. epidermidis*** |
| **Base selon l'invention sans conservateur, avec 2% de Serenibiome^{®} (Emulpro 2% Serenibiome^{®} sans conservateurs dans les figures)** | **-3,5** | **-1,1** | **-9,7** | **-6,1** |
| **Serenibiome^{®} 2%, pH=5,2** | **-2,2** | **0,1** | **-8,0** | **-6,6** |
| **Serenibiome^{®} 2%, pH=7** | **0** | **0** | **-0,4** | **-0,1** |
| **Milieu minimal, pH=5,2** | **0** | **0** | **0** | **0** |
| **Milieu minimal, pH=7** | **0** | **0** | **0** | **0** |
| **Milieu optimal, pH=5,2** | **0,6** | **0** | **1,1** | **2,1** |
| **Milieu optimal, pH=7** | **0,3** | **0** | **1,7** | **1,1** |
| **Phénoxyéthanol 0,5%** | **-3,5** | **-4,0** | **-9,7** | **-9,4** |

Le tableau 16 décrit les valeurs des réductions logarithmiques obtenues pour chaque souche en fonction du produit testé à 8h et 24h.

Les réductions logarithmiques sont calculées de la manière suivante : RL_{espèce} x = (log UFC/mL du milieu minimum + bactéries en présence du produit testé) - (log UFC/mL du milieu minimum + bactéries en absence de produit)).

**[Table 17] Bilan des résultats obtenus à 8h et 24h (Pourcentage de chaque souche dans la co-culture en présence des différents produits et témoins).**

| **Produits** | **Réduction logarithmique (RL) par espèce** | | | |
|---|---|---|---|---|
| | **8h** | | **24h** | |
| | ***S. aureus*** | ***S. epidermidis*** | ***S. aureus*** | ***S. epidermidis*** |
| **Base selon l'invention sans conservateur, avec 2% de Serenibiome^{®} (Emulpro 2% Serenibiome^{®} sans conservateurs dans les figures)** | **0%** | **100%** | **0%** | **100%** |
| **Serenibiome^{®} 2%, pH=5,2** | **0%** | **100%** | **0%** | **0%** |
| **Serenibiome^{®} 2%, pH=7** | **23,6%** | **76,4%** | **54,7%** | **45,3%** |
| **Milieu minimal, pH=5,2** | **22,1%** | **77,9%** | **96,5%** | **3,5%** |
| **Milieu minimal, pH=7** | **22,0%** | **78,0%** | **68,5%** | **31,1%** |
| **Milieu optimal, pH=5,2** | **13,4%** | **86,6%** | **74,1%** | **25,9%** |
| **Milieu optimal, pH=7** | **26,4%** | **73,6%** | **88,7%** | **11,3%** |
| **Phénoxyéthanol 0,5%** | **0%** | **0%** | **0%** | **0%** |

### IV - Conclusions

Les composés suivants ont été testés sur le modèle de co-culture S. *aureus*/*S. epidermidis.* Les effets de ces composés sur le modèle de co-culture sont résumés dans le Tableau 18 ci-dessous:

**[Table 18] Bilan des résultats obtenus à 8h et 24h (Pourcentage de chaque souche dans la co-culture en présence des différents produits et témoins).**

| | **Effet** | |
|---|---|---|
| | **Protocole GLYcoDiag (ratio 1:2 Sa:Se à t0, pH=7)** | **Protocole Solabia (ratio 1:9 Sa:Se à t0, pH=5,2 et 7)** |
| **Base selon l'invention (Emulpro placebo dans les figures)** | **Effet antibactérien sur les deux souches (conservateurs)** | **-** |
| **Base selon l'invention, avec 2% de Serenibiome^{®} (Emulpro 2% Serenibiome^{®} dans les figures)** | **Effet antibactérien sur les deux souches (conservateurs)** | **-** |
| **Base selon l'invention sans conservateur, avec 2% de Serenibiome^{®} (Emulpro 2% Serenibiome^{®} sans conservateurs dans les figures)** | **A 8h, diminution de 2 log de la souche *S. aureus.* A 24h, effet-antibactérien sur les deux souches.** | **A 8h et 24h, effet antibactérien total sur S. *aureus.* Pas d'effet sur *S. epidermidis* à 8h et diminution de 6 log à 24h.** |
| **Base selon l'invention avec 4% de Serenibiome^{®} (Emulpro 4% Serenibiome^{®} dans les figures)** | **Effet antibactérien sur les deux souches (conservateurs)** | **-** |
| **Serenibiome^{®} 2%** | **Pas d'effet sur les deux souches.** | **A 8h, effet antibactérien sur *S. aureus* et à 24h, effet anti-bactérien sur les deux souches, à pH=5,2. Pas d'effet sur les deux souches à pH=7.** |

En conclusion :
- L'effet antibactérien du Serenibiome^{®} à 2% est observé uniquement à pH=5.2, avec une cinétique plus rapide sur S. aureus par rapport à S. epidermidis, puisque l'effet est observé dès 8h. Aucun effet du Serenibiome^{®} n'est observé à pH=7. Le pH du milieu joue donc un rôle dans l'effet du Serenibiome^{®} seul à 2% sur les souches de la co-culture.
- L'effet antibactérien du Serenibiome^{®} est également observé en formulation à 2%, sans conservateurs. L'effet anti-microbien est total sur *S. aureus* à partir de 8h. Sur *S. epidermidis,* aucun effet n'est observé à 8h mais une diminution de 6 log est observée à 24h.
- La concentration de départ à t0 (103 ou 105 cfu/mL) n'a pas d'effet sur les résultats obtenus. Le ratio 1:2 *(S. aureus: S. epidermidis)* à t0, établit par GLYcoDiag, permet d'obtenir dès 8h la proportion cible souhaitée, c'est-à-dire un ratio 9:1 *(S. aureus: S. epidermidis)* en milieux de culture minimal et optimal. Le ratio 1:9 (S. *aureus: S. epidermidis) à* t0, (utilisé par Solabia), permet d'obtenir à 24h 70-90% de S. aureus et 10-30% de S. *epidermidis* en milieux de culture minimal et optimal, mais uniquement un ratio 20-30 % de S. aureus et 70-80 % de S. *epidermidis* à 8h.

### Résumé :

En résumé, cette étude *in vitro* démontre que :

### Serenibiome^{®} seul à 2 % et 4%

▪ Activité ciblée sur *S. aureus* observée à pH 5 mais pas à pH 7
▪ Pas de différence d'activité significative entre 2 et 4% de Serenibiome^{®}. Réduction de 2 log sur *S. aureus* et peu d'activité sur S. *epidermidis* à 8h
▪ A 24h, perte de la sélectivité avec activité antibactérienne très importante sur *S. aureus* et S. *epidermidis,* 8 log sur S. *Aureus* et 6 log sur *Epidermidis*

### Base de formulation selon l'invention (EMULPRO) seul sans conservateurs

▪ Pas d'activité antibactérienne à 8h mais de façon surprenante, la base de formulation selon l'invention (EMULPRO) sans actif présente une activité sur S. *aureus* et S. *epidermidis* à 24h. 7 log sur *S. aureus* et 3 log sur S. *epidermidis.*

### Base de formulation selon l'invention (EMULPRO) + 2 ou 4% SERENIBIOME^{®} sans conservateurs

▪ L'effet pH est également observé avec aucune activité antibactérienne à pH 7
▪ A pH 5.2 est retrouvée l'activité antibactérienne ciblée sur *S. Aureus* sur les formules Emulpro / Serenibiome^{®}.
▪ De façon surprenante et très intéressante, cette activité antibactérienne ciblée est maximale sur Emulpro + 2% SERENIBIOME^{®} avec une réduction plus importante sur *S. aureus* et moins importante sur *S. epidermis* : une réduction de 3.5 log sur *S. aureus* et 1 log sur S. epidermidis alors qu'avec 4% de Serenibiome^{®} nous observons une réduction de 2 log sur S. *aureus* et peu d'activité sur *S. epidermidis.*
▪ Les résultats à 24h montrent une activité antibactérienne équivalente sur toutes formules avec toujours une activité la plus importante sur la formule «Emulpro/Serenibiome^{®} 2%.

### Exemple 4 : Evaluation de la compatibilité cutanée d'un produit cosmétique sous contrôle dermatologique - patch test 20 volontaires pendant 48 heures

### 1. Objectif de l'exemple

L'objectif de cet exemple était d'évaluer la tolérance cutanée d'une composition après une application unique sur la peau de volontaires humains, dans des conditions de patch-test semi-occlusif.

### 2. Matériel et méthodes

La composition testée était un produit référencé EMULPRO LOT 250102, se présentant sous la forme d'une émulsion pour le soin du corps.

La dénomination « Emulpro » désigne la composition présentée dans le tableau 1 (Table 1), à savoir :

| **Composés** | **Fonction** | **Base selon l'invention (en % m/m)** |
|---|---|---|
| **Eau purifiée** | **Solvant** | **Qs 100** |
| **Glycérine** | **Agent humectant** | **5.00** |
| **Triglycéride d'acide caprylique et d'acide caprique;** | **Solvant huileux** | **20.00** |
| **Lauryl glucoside, myristyl glucoside et polyglyceryl-6-laurate** | **Emulsionnant** | **0.50** |
| **Gomme sclérotique** | **Agent gélifiant** | **1.00** |
| **Tocopherol** | **Anti-oxydant** | **0.30** |
| **Pentylène glycol** | **Conservateur** | **5.00** |
| **Sorbate de potassium** | **Conservateur** | **0.125** |
| **Acide citrique** | **Ajusteur de pH** | **Qs pH 5.0-5.5** |
| | **Total** | **100** |

Ici, le lot EMULPRO LOT 250102 comprend 2% de SERENIBIOME^{©}, un actif antibactérien (SERENIBIOME^{®}) qui est un mélange de propanediol et de glycolipides pour la dermatite atopique.

L'évaluation a été menée sur un panel de 23 volontaires sains (22 femmes, 1 homme), âgés de 21 à 70 ans, présentant une peau de type sensible. Les sujets ont été sélectionnés sur la base de critères d'inclusion et d'exclusion stricts, incluant notamment l'absence d'antécédents d'intolérance ou de réactions allergiques aux produits cosmétiques, l'absence de lésions cutanées, cicatrices ou tatouages sur la zone d'étude, et l'absence de traitements topiques ou systémiques pouvant interférer avec les résultats.

La composition a été appliquée pure, non diluée. Une quantité de la composition a été déposée sur un disque de papier filtre de 8 mm de diamètre (Finn Chambers^{®}) jusqu'à imprégnation. Un témoin négatif, constitué d'eau distillée appliquée sur un disque de papier filtre identique, a été préparé pour chaque sujet.

Les patchs contenant la composition à tester et le témoin négatif ont été appliqués en mode semi-occlusif sur le dos des sujets (zone scapulaire) à l'aide d'un support adhésif (Scanpor^{®}), pour une surface d'application de 50 mm². Les patchs ont été laissés en place pendant une durée de 48 heures.

Après 48 heures, les patchs ont été retirés et la zone d'étude a été rincée à l'eau distillée. Les réactions cutanées ont été évaluées visuellement par un technicien qualifié 30 minutes après le retrait des patchs.

Les réactions d'érythème (E) et d'œdème (O) ont été cotées selon une échelle numérique de 0 à 3, comme détaillé dans le Tableau 19.

**[Table 19] Échelle de cotation des réactions cutanées. Le tableau suivant présente l'échelle numérique utilisée pour coter la sévérité des réactions d'érythème et d'œdème.**

| **Score** | **Erythème (E)** | **Œdème (O)** |
|---|---|---|
| **0** | **Aucun érythème** | **Aucun œdème** |
| **0.5** | **Erythème léger** | **Œdème léger** |
| **1** | **Erythème modéré** | **Œdème modéré** |
| **2** | **Erythème vif** | **Œdème important** |
| **3** | **Erythème vif diffusant en dehors de la surface testée** | **Œdème important avec vésicules** |

Pour chaque sujet, un indice d'irritation volontaire (IIV) a été calculé en additionnant les scores d'érythème et d'œdème. Un indice d'irritation moyen (IIM) pour l'ensemble du panel a ensuite été déterminé en calculant la moyenne des IIV des 23 sujets.

La classification de la tolérance cutanée a été établie sur la base de l'IIM, une composition étant classée comme « non-irritante » pour une valeur d'IIM inférieure à 0,25.

### 3. Résultats expérimentaux

Aucune réaction cutanée n'a été observée pour le témoin négatif sur l'ensemble des 23 sujets.

Concernant la composition testée, 22 des 23 sujets n'ont présenté aucune réaction cutanée (score d'érythème et d'œdème de 0).

Un seul sujet (sujet n°11) a présenté une réaction cutanée, cotée comme un érythème léger (score E = 0.5) et une absence d'œdème (score O = 0). L'indice d'irritation volontaire (IIV) pour ce sujet était donc de 0,5.

L'indice d'irritation moyen (IIM) calculé pour la composition sur l'ensemble des 23 sujets était de 0,02. Les résultats individuels sont présentés dans le Tableau 2.

### 4. Conclusion

Les résultats de cet exemple démontrent que la composition selon l'invention présente une excellente tolérance cutanée dans les conditions de l'essai. L'indice d'irritation moyen (IIM) obtenu de 0,02 étant significativement inférieur au seuil de 0,25, la composition est classée comme non-irritante.

### Exemple 5 - Évaluation de la tolérance et de l'efficacité cosmétique en conditions d'usage sous contrôle pédiatrique

### 1. Objectif de l'exemple

L'objectif de cet exemple était d'évaluer la tolérance cutanée, l'acceptabilité cosmétique et l'efficacité d'une composition lors d'une application répétée sur la peau d'une population pédiatrique, dans des conditions normales d'utilisation et sous le contrôle d'un pédiatre.

### 2. Matériel et méthodes

La composition testée était un produit référencé EMULPRO (réf.: ZT9325 - Lot: 250102).

La dénomination « Emulpro » désigne la composition présentée dans le tableau 1 (Table 1), à savoir :

| **Composés** | **Fonction** | **Base selon l'invention (en % m/m)** |
|---|---|---|
| **Eau purifiée** | **Solvant** | **Qs 100** |
| **Glycérine** | **Agent humectant** | **5.00** |
| **Triglycéride d'acide caprylique et d'acide caprique;** | **Solvant huileux** | **20.00** |
| **Lauryl glucoside, myristyl glucoside et polyglyceryl-6-laurate** | **Emulsionnant** | **0.50** |
| **Gomme sclérotique** | **Agent gélifiant** | **1.00** |
| **Tocopherol** | **Anti-oxydant** | **0.30** |
| **Pentylène glycol** | **Conservateur** | **5.00** |
| **Sorbate de potassium** | **Conservateur** | **0.125** |
| **Acide citrique** | **Ajusteur de pH** | **Qs pH 5.0-5.5** |
| | **Total** | **100** |

Ici, le lot EMULPRO LOT 250102 comprend 2% de SERENIBIOME^{©}, un actif antibactérien (SERENIBIOME^{®}) qui est un mélange de propanediol et de glycolipides pour la dermatite atopique.

L'évaluation a été menée sur un panel de 25 sujets sains, âgés de 5 à 36 mois (âge moyen : 23,9 mois), incluant 10 filles (40%) et 15 garçons (60%). Les caractéristiques cutanées de la population étaient les suivantes : 100% des sujets (25/25) présentaient une peau sensible. Parmi eux, 12 sujets (48%) avaient la peau sèche, 4 sujets (16%) la peau très sèche, et 9 sujets (36%) la peau normale. Un sujet (4%) présentait des antécédents d'atopie. Les caractéristiques de la population sont les suivantes :
- **A)** Répartition par sexe (40% de filles, 60% de garçons).
- **B)** Nature de la peau (36% normale, 48% sèche, 16% très sèche).
- **C)** Sensibilité cutanée (100% des sujets).
- **D)** Antécédents d'atopie (4% des sujets).

La composition a été appliquée une fois par jour, pendant 3 semaines consécutives, sur la peau du corps des sujets, dans leurs conditions normales d'utilisation à domicile.

La tolérance cutanée a été évaluée par un pédiatre investigateur à la fin de la période d'étude de 3 semaines, par observation de tout signe clinique anormal. L'acceptabilité cosmétique et l'efficacité perçue ont été évaluées au moyen de questionnaires d'auto-évaluation remplis par les parents ou tuteurs légaux des sujets à la fin de la période d'étude.

### 3. Résultats expérimentaux

Après 3 semaines d'application, aucun signe clinique anormal n'a été observé par le pédiatre investigateur sur l'ensemble des 25 sujets. Le parent d'un sujet (sujet n°26, avec une peau sensible et sèche) a rapporté l'apparition occasionnelle d'une rougeur de légère intensité durant quelques minutes après certaines applications. Cette manifestation isolée n'a nécessité aucune modification du protocole d'application. Au total, une seule réaction (rougeur) a été notée pour un seul sujet sur l'ensemble de l'étude. Aucune réaction n'a été considérée comme "pertinente" ou comme un effet indésirable lié au produit.

Les évaluations subjectives par les parents ont montré des résultats favorables pour l'ensemble des propriétés évaluées. Les principaux résultats sont les suivants :
- Le produit a été jugé adapté à la peau sensible par 100% des parents.
- Le produit a été jugé idéal pour un usage fréquent par 100% des parents.
- Le produit a été perçu comme laissant la peau douce et sans tiraillements par 100% des parents.
- Le produit a été perçu comme procurant une sensation de confort et de bien-être par 100% des parents.
- Le produit a été perçu comme hydratant intensément la peau par 96% des parents.
- Le produit a été perçu comme soulageant les démangeaisons par 92% des parents.
- Une perception globale positive du produit a été rapportée par 96% des parents.
- Une intention d'achat du produit a été exprimée par 92% des parents.

### 4. Conclusion

Les résultats de cet exemple démontrent que la composition testée présente une excellente tolérance cutanée lorsqu'elle est appliquée quotidiennement pendant 3 semaines sur une population pédiatrique à peau sensible, y compris sèche à très sèche. De plus, la composition a démontré une haute acceptabilité cosmétique et une efficacité perçue, notamment en termes d'hydratation, d'apaisement et d'adéquation avec les peaux sensibles.

### Exemple 6 - Évaluation du potentiel d'irritation cutanée in vitro sur explants de peau humaine

### 1. Objectif de l'exemple

L'objectif de cet exemple était d'évaluer le potentiel d'irritation cutanée d'une composition par une mesure de la viabilité cellulaire sur des explants de peau humaine fraîche.

### 2. Matériel et méthodes

La composition testée était un produit référencé EMULPRO - ZT9325 (Lot 250102), se présentant sous la forme d'une crème blanche, classée comme un produit de soin pour le visage et le corps pour bébés.

La dénomination « Emulpro » désigne la composition présentée dans le tableau 1 (Table 1), à savoir :

| **Composés** | **Fonction** | **Base selon l'invention (en % m/m)** |
|---|---|---|
| **Eau purifiée** | **Solvant** | **Qs 100** |
| **Glycérine** | **Agent humectant** | **5.00** |
| **Triglycéride d'acide caprylique et d'acide caprique;** | **Solvant huileux** | **20.00** |
| **Lauryl glucoside, myristyl glucoside et polyglyceryl-6-laurate** | **Emulsionnant** | **0.50** |
| **Gomme sclérotique** | **Agent gélifiant** | **1.00** |
| **Tocopherol** | **Anti-oxydant** | **0.30** |
| **Pentylène glycol** | **Conservateur** | **5.00** |
| **Sorbate de potassium** | **Conservateur** | **0.125** |
| **Acide citrique** | **Ajusteur de pH** | **Qs pH 5.0-5.5** |
| | **Total** | **100** |

Ici, le lot EMULPRO LOT 250102 comprend 2% de SERENIBIOME^{©}, un actif antibactérien (SERENIBIOME^{®}) qui est un mélange de propanediol et de glycolipides pour la dermatite atopique.

Le système de test était constitué de disques de peau humaine fraîche (explants) obtenus suite à des interventions de chirurgie plastique et maintenus en survie dans des boîtes de Pétri avec un milieu de survie.

La composition testée a été appliquée pure, à une concentration de 100%, par voie topique sur les explants de peau. Les explants traités ont été incubés pendant une durée de 20 ± 2 heures à une température de +32 ± 1°C. Un témoin positif, constitué d'une solution de SDS à 50 ng/mL, a été inclus pour valider la sensibilité du système de test.

Après la période d'incubation, la viabilité cellulaire de l'épiderme a été évaluée par un test au MTT (bromure de 3-(4,5-diméthylthiazol-2-yl)-2,5-diphénylté-trazolium), un marqueur dépendant de l'activité métabolique des cellules. Une vérification de l'absence d'interférence entre la composition et le réactif MTT a été effectuée. Le potentiel d'irritation a été classifié sur la base du pourcentage de viabilité cellulaire obtenu par rapport à un témoin négatif. Une composition est classée comme « non-irritante » pour une viabilité cellulaire supérieure ou égale à 50%, et comme « irritante » pour une viabilité inférieure à 50%.

### 3. Résultats expérimentaux

La validité de l'essai a été confirmée par l'obtention d'une viabilité cellulaire de 31% (± 7%) pour le témoin positif (SDS), ce qui est inférieur au seuil de 50%. Il a également été vérifié que la composition testée n'interférait pas avec l'essai MTT.

Pour la composition testée appliquée à 100%, les pourcentages de viabilité cellulaire mesurés sur des réplicats individuels étaient de 108%, 113% et 114%.

La viabilité cellulaire moyenne pour la composition a été déterminée à 112%.

Sur la base de ce résultat, la composition a été classée comme « non-irritante ».

### 4. Conclusion

Les résultats de cet exemple démontrent que la composition présente une excellente tolérance cutanée in vitro. La viabilité cellulaire de 112%, très supérieure au seuil de 50%, confirme l'absence de potentiel irritant de la composition par contact direct sur la peau.

### Exemple 7 - Évaluation du potentiel d'irritation oculaire in vitro par relargage du rouge neutre

### 1. Objectif de l'exemple

L'objectif de cet exemple était d'évaluer le potentiel d'irritation oculaire d'une composition par une mesure de sa cytotoxicité sur une monocouche de fibroblastes de cornée de lapin.

### 2. Matériel et méthodes

La composition testée était un produit référencé EMULPRO - ZT9325 (Lot 250102), se présentant sous la forme d'une crème blanche.

La dénomination « Emulpro » désigne la composition présentée dans le tableau 1 (Table 1), à savoir :

| **Composés** | **Fonction** | **Base selon l'invention (en % m/m)** |
|---|---|---|
| **Eau purifiée** | **Solvant** | **Qs 100** |
| **Glycérine** | **Agent humectant** | **5.00** |
| **Triglycéride d'acide caprylique et d'acide caprique;** | **Solvant huileux** | **20.00** |
| **Lauryl glucoside, myristyl glucoside et polyglyceryl-6-laurate** | **Emulsionnant** | **0.50** |
| **Gomme sclérotique** | **Agent gélifiant** | **1.00** |
| **Tocopherol** | **Anti-oxydant** | **0.30** |
| **Pentylène glycol** | **Conservateur** | **5.00** |
| **Sorbate de potassium** | **Conservateur** | **0.125** |
| **Acide citrique** | **Ajusteur de pH** | **Qs pH 5.0-5.5** |
| | **Total** | **100** |

Ici, le lot EMULPRO LOT 250102 comprend 2% de SERENIBIOME^{©}, un actif antibactérien (SERENIBIOME^{®}) qui est un mélange de propanediol et de glycolipides pour la dermatite atopique.

Le système de test était une monocouche de fibroblastes de cornée de lapin (lignée cellulaire SIRC).

La composition a été diluée dans un véhicule (NaCl à 0,9%) pour obtenir des concentrations de 10%, 25%, 50% et 100%. Les cellules SIRC ont été mises en contact avec ces différentes concentrations de la composition pendant une durée de 60 secondes. Un témoin positif (SDS à 0,01%, 0,05% et 0,2%) et un témoin négatif (véhicule seul) ont été inclus pour valider l'essai.

Après la période de contact, la viabilité cellulaire a été évaluée par la méthode du relargage du rouge neutre. L'absorbance (densité optique, DO) a été mesurée pour déterminer le pourcentage de mortalité cellulaire à chaque concentration. Le potentiel de cytotoxicité a été déterminé en calculant la concentration entraînant 50% de mortalité cellulaire (IC50). La classification a été établie sur la base des critères suivants :
- **Cytotoxicité négligeable** : IC50 > 50% et pourcentage de mortalité observé à 50% de concentration ≤ 20%.
- **Cytotoxicité peu importante** : IC50 > 50% et pourcentage de mortalité observé à 50% de concentration > 20% et < 50%.
- **Cytotoxicité modérée :** IC50 > 25% et ≤ 50%.
- **Cytotoxicité importante** : IC50 ≤ 25%.

### 3. Résultats expérimentaux

La validité de l'essai a été confirmée, la densité optique du témoin négatif étant de 1,268 (valeur attendue ≥ 0,8) et l'IC50 du témoin positif (SDS) étant comprise entre 0,01% et 0,2%.

Pour la composition testée, les résultats suivants ont été obtenus :
- Le pourcentage de mortalité cellulaire observé à une concentration de 50% était de 2%.
- La concentration IC50 a été déterminée comme étant supérieure à 50%.

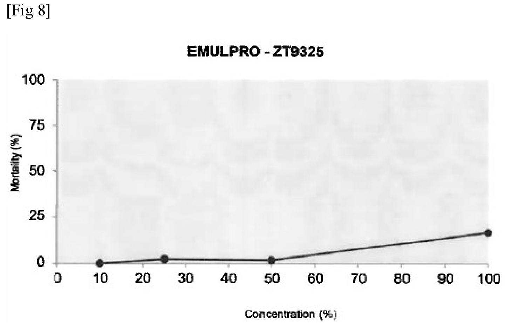

Conformément aux critères de classification, la composition a été classée comme présentant une **cytotoxicité négligeable.** Ces résultats sont illustrés à la Figure 8.

### 4. Conclusion

Les résultats de cet exemple démontrent que la composition présente une excellente tolérance oculaire in vitro. Le classement en « cytotoxicité négligeable » indique que la composition est non-irritante pour l'œil dans les conditions de l'essai.

### Exemple 8 - Évaluation de la tolérance, de l'efficacité perçue et de l'acceptabilité en conditions d'usage sous contrôle dermatologique

### 1. Objectif de l'exemple

L'objectif de cet exemple était d'évaluer la tolérance cutanée, l'efficacité perçue et l'acceptabilité d'une composition lors d'une application répétée pendant 28 jours sur une population adulte, dans des conditions normales d'utilisation et sous contrôle dermatologique.

### 2. Matériel et méthodes

La composition testée était un produit référencé EMULPRO (réf.: ZT9325 - Lot: 250102), se présentant sous la forme d'une crème blanche.

La dénomination « Emulpro » désigne la composition présentée dans le tableau 1 (Table 1), à savoir :

| **Composés** | **Fonction** | **Base selon l'invention (en % m/m)** |
|---|---|---|
| **Eau purifiée** | **Solvant** | **Qs 100** |
| **Glycérine** | **Agent humectant** | **5.00** |
| **Triglycéride d'acide caprylique et d'acide caprique;** | **Solvant huileux** | **20.00** |
| **Lauryl glucoside, myristyl glucoside et polyglyceryl-6-laurate** | **Emulsionnant** | **0.50** |
| **Gomme sclérotique** | **Agent gélifiant** | **1.00** |
| **Tocopherol** | **Anti-oxydant** | **0.30** |
| **Pentylène glycol** | **Conservateur** | **5.00** |
| **Sorbate de potassium** | **Conservateur** | **0.125** |
| **Acide citrique** | **Ajusteur de pH** | **Qs pH 5.0-5.5** |
| | **Total** | **100** |

Ici, le lot EMULPRO LOT 250102 comprend 2% de SERENIBIOME^{©}, un actif antibactérien (SERENIBIOME^{®}) qui est un mélange de propanediol et de glycolipides pour la dermatite atopique.

L'évaluation a été menée sur un panel de 20 volontaires (14 femmes, 6 hommes), âgés de 23 à 70 ans (âge moyen : 57 ans). La totalité du panel (100%) présentait une peau du corps sèche à très sèche, une peau sensible et une peau à tendance atopique.

La composition a été appliquée matin et soir pendant 28 jours consécutifs sur l'ensemble du visage et du corps. Il a été demandé aux volontaires d'utiliser la composition en remplacement de leur crème visage et/ou corps habituelle.

### Analyse des résultats

Un examen dermatologique a été réalisé par télé-expertise (sur la base de photographies) au début de l'essai (J0) et à la fin (J28). Les signes cliniques d'érythème, de sécheresse, de desquamation et d'hyperkératose ont été cotés sur une échelle de 0 (absence) à 10 (sévère). La significativité statistique de l'évolution des scores entre J0 et J28 a été évaluée par un test non paramétrique de Wilcoxon.

Les sensations d'inconfort ou d'intolérance ressenties par les volontaires au cours des 28 jours d'utilisation ont été recueillies et analysées.

Un questionnaire a été complété par les volontaires à J28 pour évaluer leur appréciation de la composition et de son efficacité.

### 3. Résultats expérimentaux

Sur les 20 volontaires ayant débuté l'essai, 19 ont été inclus dans l'analyse de la tolérance (un volontaire n'ayant pas pu se rendre à la visite finale) et 20 ont été inclus dans l'analyse de l'acceptabilité et de l'efficacité perçue.

L'analyse comparative des scores cliniques entre J0 et J28 a montré l'absence de différence statistiquement significative pour l'érythème, la sécheresse, la desquamation et l'hyperkératose (p = 1,000 pour chaque signe). Sur la base de ces examens, la tolérance clinique cutanée de la composition a été jugée bonne.

Aucun des 19 volontaires présents aux deux visites n'a rapporté de sensation d'inconfort ou de réaction d'intolérance au cours des 28 jours d'utilisation. Le score global de symptomatologie fonctionnelle pour le panel était de 0,0, correspondant à une tolérance fonctionnelle jugée très bonne.

En considérant la tolérance clinique et fonctionnelle, la tolérance cutanée globale de la composition au cours des 28 jours d'utilisation a été jugée très bonne.

Les résultats du questionnaire à J28 ont montré une appréciation très favorable du produit. Les principaux résultats sont les suivants (en % de volontaires en accord ("Tout à fait d'accord" + "Plutôt d'accord") :
- Le produit laisse la peau hydratée après application : 100%
- La peau est douce : 95%
- La peau est hydratée immédiatement : 95%
- Le produit apporte un effet apaisant : 95%
- Le produit calme et soulage les sensations d'inconfort : 95%
- La peau est nourrie : 95%:

### 4. Conclusion

Les résultats de cet exemple démontrent que la composition présente une excellente tolérance cutanée, clinique et fonctionnelle, lorsqu'elle est appliquée deux fois par jour pendant 28 jours sur une population adulte ayant une peau sensible, sèche et à tendance atopique. De plus, la composition a démontré une haute acceptabilité cosmétique et une efficacité perçue, notamment en termes d'hydratation, de douceur et d'apaisement.

### Exemple 9 - Évaluation de la tolérance et de l'efficacité cosmétique en conditions d'usage sous contrôle dermatologique et gynécologique

### 1. Objectif de l'exemple

L'objectif de cet exemple était d'évaluer la tolérance cutanée, l'acceptabilité cosmétique et l'efficacité perçue d'une composition lors d'une application répétée sur la zone intime externe d'une population adulte féminine, dans des conditions normales d'utilisation et sous contrôle dermatologique et gynécologique.

### 2. Matériel et méthodes

La composition testée était un produit référencé EMULPRO INTIMATE CREAM (réf.: EIC-250906 - Lot: 250296).

La dénomination « Emulpro » désigne la composition présentée dans le tableau 1 (Table 1), à savoir :

| **Composés** | **Fonction** | **Base selon l'invention (en % m/m)** |
|---|---|---|
| **Eau purifiée** | **Solvant** | **Qs 100** |
| **Glycérine** | **Agent humectant** | **5.00** |
| **Triglycéride d'acide caprylique et d'acide caprique;** | **Solvant huileux** | **20.00** |
| **Lauryl glucoside, myristyl glucoside et polyglyceryl-6-laurate** | **Emulsionnant** | **0.50** |
| **Gomme sclérotique** | **Agent gélifiant** | **1.00** |
| **Tocopherol** | **Anti-oxydant** | **0.30** |
| **Pentylène glycol** | **Conservateur** | **5.00** |
| **Sorbate de potassium** | **Conservateur** | **0.125** |
| **Acide citrique** | **Ajusteur de pH** | **Qs pH 5.0-5.5** |
| | **Total** | **100** |

Ici, la crème contient de l'huile de cranberry (0,5000% en % m/m) pour apaiser la zone intime fragilisée et un mélange d'acides hyaluroniques de différents poids moléculaires (SODIUM HYALURONATE à poids moléculaire élevé : 0,2500% en % m/m / SODIUM HYALURONATE à faible poids moléculaire : 0,2500% en % m/m) pour renforcer l'hydratation.

L'évaluation a été menée sur un panel de 20 sujets féminins adultes, âgés de 18 à 70 ans. La totalité du panel (100%) présentait une peau de la zone intime sensible et rapportait des symptômes de sécheresse vaginale, d'inconfort ou de sensibilité vulvaire. Les caractéristiques de la population sont les suivantes : • A) Sensibilité de la peau de la zone intime (100% des sujets). • B) Nature de la peau de la zone intime (40% normale, 55% sèche, 5% très sèche).

La composition a été appliquée une fois par jour pendant 4 semaines consécutives sur la zone intime externe. L'application a été réalisée par les sujets euxmêmes, à domicile, dans leurs conditions normales d'utilisation.

### Analyse des résultats

Des examens cliniques ont été réalisés par un dermatologue et un gynécologue au début de l'essai (J1) et à la fin (J29) pour évaluer l'apparition de tout signe clinique anormal.

Les sensations d'inconfort ou d'irritation ressenties par les sujets au cours de l'étude ont été recueillies.

Un questionnaire a été complété par les sujets à la fin de la période d'étude pour évaluer leur appréciation de la composition et de son efficacité.

### 3. Résultats expérimentaux

Sur les 24 sujets initialement recrutés, 20 ont complété l'étude et ont été inclus dans l'analyse des résultats.

Après 4 semaines d'application, aucun signe clinique anormal n'a été observé par le dermatologue et le gynécologue investigateurs sur l'ensemble des 20 sujets. De plus, aucun sujet n'a rapporté avoir ressenti ou observé de réaction cutanée d'irritation ou d'inconfort au cours de l'étude.

Les résultats du questionnaire d'auto-évaluation ont montré une appréciation très favorable du produit. Les principaux résultats sont les suivants :
- 100% des sujets ont trouvé que la texture était agréable à appliquer et que le produit était absorbé rapidement sans laisser de résidu gras.
- 100% des sujets ont trouvé que le produit ne provoque aucun effet indésirable.
- 95% des sujets ont trouvé que la peau était immédiatement hydratée.
- 95% des sujets ont trouvé que le produit procurait un soulagement immédiat de l'inconfort et des démangeaisons.
- 95% des sujets ont trouvé que le produit était adapté aux peaux sensibles.
- 95% des sujets ont trouvé que le produit aidait à préserver l'équilibre naturel de la flore intime.
- 90% des sujets recommanderaient le produit à d'autres femmes.

### 4. Conclusion

Les résultats de cet exemple démontrent que la composition présente une excellente tolérance cutanée et gynécologique lorsqu'elle est appliquée quotidiennement pendant 4 semaines sur une population féminine ayant une zone intime sensible et sujette à l'inconfort. De plus, la composition a démontré une haute acceptabilité cosmétique et une efficacité perçue, notamment en termes d'hydratation, de soulagement de l'inconfort et d'adéquation avec les peaux sensibles.

## Revendications

1. Base de formulation cosmétique ou pharmaceutique, sous forme d'une émulsion gélifiée huile dans eau, comprenant :
• De l'eau ;
• De la glycérine ;
• Des triglycérides d'acide caprylique et d'acide caprique ;
• Au moins un tensioactif ;
• Au moins un gélifiant ;
• entre 1 et 10% de pentylène glycol en poids total de ladite base de formulation ;
• entre 0,01 et 0,25% de sorbate de potassium en poids total de ladite base de formulation.

2. Base de formulation cosmétique ou pharmaceutique selon la revendication précédente, ledit au moins un tensioactif étant choisi parmi :
• Un mélange de lauryl glucoside, de myristyl glucoside et de polyglyceryl-6-laurate ;
• Une mélange d'oléate de polyglycéryle-6 et d'olivate de potassium ;
• Du polyglyceryl-2-dipolyhydroxystearate ;
• Du polyglyceryl-3-diisostearate ;
• Des phospholipides hydrogénés ;
• Du myristate de polyglycéryle-10 ;
• Du surfactine sodique ;
• Un mélange d'alcool cétéarylique et de glucoside de cétéaryle ;
• Des extraits de graines de soja ;
• De la lécithine ;
• Du polysorbate 80 ;
• De l'oléate de sorbitan.

3. Base de formulation cosmétique ou pharmaceutique selon l'une quelconque des revendications précédentes, comprenant entre 3% et 7% de pentylène glycol, de préférence 5%, en poids total de ladite base de formulation.

4. Base de formulation cosmétique ou pharmaceutique selon l'une quelconque des revendications précédentes, comprenant entre 0,1 et 0,15% de sorbate de potassium, de préférence 0,125%, en poids total de ladite base de formulation.

5. Base de formulation cosmétique ou pharmaceutique selon l'une quelconque des revendications précédentes, comprenant entre 3% et 8% de glycérine, de préférence 5%, en poids total de ladite base de formulation.

6. Base de formulation cosmétique ou pharmaceutique, selon l'une quelconque des revendications précédentes, comprenant entre 10% et 30% de triglycérides d'acide caprylique et d'acide caprique, de préférence 20%, en poids total de ladite base de formulation.

7. Base de formulation cosmétique ou pharmaceutique l'une quelconque des revendications précédentes, ledit au moins un tensioactif comprenant entre 0,024 et 0,28% de lauryl glucoside, de préférence entre 0,12% et 0,14%, en poids total de ladite base de formulation.

8. Base de formulation cosmétique ou pharmaceutique l'une quelconque des revendications précédentes, ledit au moins un tensioactif comprenant entre 0,01 et 0,12% de de myristyl glucoside, de préférence entre 0,05% et 0,06%, en poids total de ladite base de formulation.

9. Base de formulation cosmétique ou pharmaceutique l'une quelconque des revendications précédentes, ledit au moins un tensioactif comprenant entre 0,0235 % et 0,245% de polyglyceryl-6-laurate, de préférence entre 0,1175% et 0,1225, en poids total de ladite base de formulation.

10. Base de formulation cosmétique ou pharmaceutique selon l'une quelconque des revendications précédentes, comprenant entre 0,1% et 2% de gomme sclérotique, de préférence 1%, en poids total de ladite base de formulation.

11. Base de formulation cosmétique ou pharmaceutique selon l'une quelconque des revendications précédentes, ledit au moins un gélifiant étant choisi parmi : gomme sclérotique, gomme de xanthane, cellulose microcristalline, agar, carraghénanes, pectines, guar, caroube, arabique, gélatine, collagène, cellulose, amidons, chitosan, alginates, argiles.

12. Base de formulation cosmétique ou pharmaceutique selon l'une quelconque des revendications précédentes, consistant en :
• 5% glycérine, en poids total de ladite base de formulation;
• 20% de triglycérides d'acide caprylique et d'acide caprique, en poids total de ladite base de formulation ;
• 0,50% d'un mélange d'eau, de lauryl glucoside, de myristyl glucoside et de polyglyceryl-6-laurate, en poids total de ladite base de formulation, ledit mélange comprenant : entre 24 et 28% du mélange de lauryl glucoside, entre 10 et 12% du mélange de myristyl glucoside, entre 23,5 et 24,5 du mélange de polyglyceryl-6 laurate et entre 30 et 45% du mélange d'eau ;
• 1% de gomme sclérotique, en poids total de ladite base de formulation ;
• 5% de pentylène glycol, en poids total de ladite base de formulation ;
• 0,125% de sorbate de potassium, en poids total de ladite base de formulation ;
• De l'eau en quantité suffisante pour compléter la base de formulation ;
• De l'acide citrique en quantité suffisante pour obtenir un pH compris entre 5 et 5,5.

13. Utilisation de la base de formulation cosmétique ou pharmaceutique selon l'une quelconque des revendications 1 à 12, pour la préparation d'une formulation cosmétique ou pharmaceutique contenant au moins un principe actif d'indication pharmaceutique ou cosmétique.

14. Dispositif médical comprenant une base de formulation selon l'une quelconque des revendications 1 à 12, ladite base de formulation étant destinée à être mélangée avec au moins un principe actif d'indication cosmétique ou pharmaceutique.

15. Dispositif médical selon la revendication précédente, ledit dispositif médical étant sélectionné parmi :
• un dispositif topique, de préférence un pansement, un patch ou une compresse ;
• un dispositif injectable, de préférence une seringue préremplie ou un implant injectable ;
• un dispositif implantable, de préférence un implant biodégradable, ou un stent, ou un cathéter ;
• un dispositif ophtalmique, nasal, auriculaire ou vaginal,
• un dispositif gastro-intestinal ou buccal.
